# EUROPEAN PATENT SPECIFICATION

(11) **EP 3 057 986 B1**
(45) Date of publication and mention of the grant of the patent: **27.12.2017**
(21) Application number: 14854582.5
(22) Date of filing: 17.10.2014
(51) Int. Cl.: C07K 14/705, C07K 16/28, C07K 14/47, C07K 14/735

(54) **CHIMERIC RECEPTOR THAT TRIGGERS ANTIBODY-DEPENDENT CELL CYTOTOXICITY AGAINST MULTIPLE TUMORS**
CHIMÄRER REZEPTOR ZUR AUSLÖSUNG VON ANTIKÖRPERABHÄNGIGER ZELLENZYTOTOXIZITÄT GEGEN MEHRERE TUMOREN
RÉCEPTEUR CHIMÉRIQUE QUI DÉCLENCHE UNE CYTOTOXICITÉ DÉPENDANTE DE LA PRÉSENCE D'ANTICORPS CONTRE DE MULTIPLES TUMEURS

(30) Priority: 17.10.2013 US 201361892218 P; 18.07.2014 US 201462026243 P
(43) Date of publication of application: 24.08.2016
(73) Proprietor: National University of Singapore, Singapore 119077 (SG); St. Jude Children's Research Hospital, Memphis, TN 38105 (US)
(72) Inventor: CAMPANA, Dario, Singapore 119077 (SG); KUDO, Ko, Singapore 119077 (SG)
(74) Representative: Potter Clarkson LLP
(86) International application number: PCT/US2014/060999
(87) International publication number: WO 2015/058018

(56) References cited:
- WO-A1-2016/040441
- US-A1- 2011 123 440
- US-A1- 2012 213 783
- US-A1- 2013 225 668
- US-A1- 2013 266 551
- K. KUDO ET AL: "T Lymphocytes Expressing a CD16 Signaling Receptor Exert Antibody-Dependent Cancer Cell Killing", CANCER RESEARCH, vol. 74, no. 1, 1 January 2014 (2014-01-01), pages 93-103, XP055222596, ISSN: 0008-5472, DOI: 10.1158/0008-5472.CAN-13-1365
- DATABASE UNIPROTKB [Online] 18 September 2013 'FULL=LOW AFFINITY IMMUNOGLOBULIN GAMMA FC REGION RECEPTOR III-A;', XP055334803 Retrieved from UNIPROT Database accession no. P08637
- DATABASE UNIPROTKB [Online] 29 May 2013 XP055334815 Retrieved from UNIPROT Database accession no. M9MML0

## Description

### FIELD OF THE PRESENT DISCLOSURE

The present disclosure is directed to a chimeric receptor that binds the Fc portion of human immunoglobulin and delivers activation signals. The chimeric receptor of the present disclosure comprises the high-affinity V158 *FCGR3A* variant, the hinge and transmembrane domains of CD8α, and the signaling domains of CD3ζ and 4-1BB. The chimeric receptor of the present disclosure has a high affinity for Rituximab, Trastuzumab, hu14.18K322A, and other therapeutic antibodies, making it useful for augmenting the efficacy of antibody therapy against various cancers.

### BACKGROUND OF THE PRESENT DISCLOSURE

Immunotherapy is a promising option for cancer treatment because of its potential to evade genetic and cellular mechanisms of drug resistance, and to target tumor cells while sparing normal tissues. T-lymphocytes can exert major anti-tumor effects as demonstrated by results of allogeneic hematopoietic stem cell transplantation (HSCT) for hematologic malignancies, where T-cell-mediated graft-versus-host disease (GvHD) is inversely associated with disease recurrence, and immunosuppression withdrawal or infusion of donor lymphocytes can contain relapse Weiden et al., N. Engl. J. Med. 1979; 300(19):1068-1073; Porter et al., N. Engl. J. Med. 1994; 330(2):100-106; Kolb et al., Blood 1995; 86(5):2041-2050; Slavin et al., Blood 1996; 87(6):2195-2204; Appelbaum, Nature 2001; 411(6835):385-389. The reactivity of T lymphocytes can be skewed towards cancer cells by the expression of chimeric signaling receptors with antibody recognition properties: ligation of the cognate target results in T-cell activation and triggers cytotoxicity Eshhar et al., Proc. Natl. Acad. Sci. USA. 1993; 90(2):720-724; Geiger et al., J. Immunol. 1999; 162(10):5931-5939; Brentjens et al., Nat. Med. 2003; 9(3):279-286; Cooper et al., Blood 2003; 101(4):1637-1644; Imai et al., Leukemia 2004; 18:676-684. Recent results of clinical trials with infusions of chimeric receptor-expressing autologous T lymphocytes provided compelling evidence of their clinical potential Pule et al., Nat. Med. 2008; 14(11):1264-1270; Porter et al., OncLive 2011; 25;365(8):725-733; Brenjens et al., Blood 2011; 118(18):4817-4828; Till et al., Blood 2012; 119(17):3940-3950; Kochenderfer et al., Blood 2012; 119(12):2709-2720; Brentjens et al., Sci. Transl. Med. 2013; 5(177):177ra138.

Another approach to cancer immunotherapy is the administration of monoclonal antibodies which can exert cytotoxicity through a variety of mechanisms including induction of pro-apoptotic signals, complement fixation and antibody-dependent cell cytotoxicity (ADCC) Yu et al., N. Engl. J. Med. 2010; 363(14):1324-1334; Ferris et al., J. Clin. Oncol. 2010; 28(28):4390-4399; Maloney, N. Engl. J. Med. 2012; 366(21):2008-2016; Scott et al., Nat. Rev. Cancer 2012; 12(4):278-287; Weiner et al., Cell 2012; 148(6):1081-1084; Galluzzi et al., Oncoimmunology 2012; 1(1):28-37. A major role is played by the latter mechanism, which results from the engagement of Fc receptors (FcγR) expressed on the surface of natural killer (NK cells) and other cells, such as neutrophils and macrophages, by the Fc portion of the antibody Nimmerjahn et al., Nat. Rev. Immunol. 2008; 8(1):34-47. Polymorphisms of FcγR genes can have marked functional consequences which, in turn, influence response to antibody treatment. To this end, allotypes of the gene coding FcγRIIIa (*FCRG3A* or *CD16*), expressed by NK cells, can result in receptors having either a phenylalanine (F) or a valine (V) residue at position 158; CD16 158 V/V, which occurs in a minority of individuals, has higher Fc binding and is associated with increased tumor cell killing and superior responses in patients Ferris et al., J. Clin. Oncol. 2010; 28(28):4390-4399; Koene et al., Blood 1997; 90(3):1109-1114; Cartron et al., Blood 2002; 99(3):754-758; Weng et al., J. Clin. Oncol. 2003; 21(21):3940-3947; Dall'Ozzo et al., Cancer Res. 2004; 64(13):4664-4669; Hatjiharissi et al., Blood 2007; 110(7):2561-2564; Musolino et al., J. Clin. Oncol. 2008; 26(11): 1789-1796; Bibeau et al., J. Clin. Oncol. 2009; 27(7): 1122-1129; Ahlgrimm et al., Blood 2011; 118(17):4657-4662; Veeramani et al., Blood 2011; 118(12):3347-3349.

US 2013/266551 A1 relates to a chimeric receptor capable of signaling both a primary and a co-stimulatory pathway, allowing activation of the co-stimulatory pathway without binding to the natural ligand. The cytoplasmic domain of the receptor contains a portion of the 4-1BB signaling domain. US 2011/123440 A1 relates to altered antibody Fc regions and uses thereof. Specifically, the altered Fc regions in that document have one or more amino acid substitutions relative to the sequence of a corresponding unaltered (wild-type or parent) Fc region, and have one or more properties that differ from a corresponding unaltered Fc region.

There is a great need to develop new approaches and new agents for therapy of cancer.

### SUMMARY OF THE PRESENT DISCLOSURE

The present invention is defined in the accompanying claims and relates to chimeric receptors, isolated polynucleotides encoding said chimeric receptors, vectors comprising said isolated polynucleotides, host cells expressing said chimeric receptors, pharmaceutical compositions comprising said isolated polynucleotides, vectors, or host cells, and T lymphocytes and NK cells expressing said chimeric receptors. Embodiments of the invention are described in the following numbered paragraphs:
(1). A chimeric receptor comprising: (a) an extracellular ligand-binding domain of F158 *FCGR3A* or the V158 *FCGR3A* variant; (b) a hinge and transmembrane domains of CD8α; and (c) a cytoplasmic domain comprising a 4-1BB signaling domain and a CD3ζ signaling domain.
(2). The chimeric receptor of paragraph 1, wherein the chimeric receptor further comprises a signal peptide of CD8α; and/or, optionally wherein the extracellular ligand-binding domain of F158 *FCGR3A* and the V158 *FCGR3A* variant consists of the amino acid sequence of SEQ ID NO: 16 and SEQ ID NO: 2, respectively; and/or optionally wherein the hinge and transmembrane domains of CD8α consist of the amino acid sequence of SEQ ID NO: 3; and/or optionally wherein the 4-1BB signaling domain consists of the amino acid sequence of SEQ ID NO: 4; and/or, optionally wherein the CD3ζ signaling domain consists of the amino acid sequence of SEQ ID NO: 5; and/or optionally wherein the signal peptide of CD8α consists of the amino acid sequence of SEQ ID NO: 6.
(3). The chimeric receptor of paragraph 1, which comprises the amino acid sequence of residues 22-436 of SEQ ID NO: 1 or SEQ ID NO:15, or which comprises the amino acid sequence of SEQ ID NO: 1 or SEQ ID NO:15.
(4). An isolated polynucleotide, comprising a nucleotide sequence encoding the chimeric receptor of any one of paragraphs 1-3.
5. The isolated polynucleotide of paragraph 4, which comprises the nucleotide sequence of SEQ ID NO: 10 or SEQ ID NO: 18; and/or optionally wherein the nucleotide sequence of SEQ ID NO: 11; and/or optionally wherein the nucleotide sequence of SEQ ID NO: 12; and/or optionally wherein the nucleotide sequence of SEQ ID NO: 13; and/or optionally wherein the nucleotide sequence of SEQ ID NO: 14.
6. The isolated polynucleotide of paragraph 4, which comprises the nucleotide sequence of SEQ ID NO: 9 or SEQ ID NO: 17.
7. A pharmaceutical composition comprising the polynucleotide of any one of paragraphs 4-6 and a pharmaceutically acceptable carrier or excipient.
8. A vector comprising the polynucleotide of any one of paragraphs 4-6; optionally wherein the polynucleotide is operatively linked to at least one regulatory element for expression of the chimeric receptor encoded by the polynucleotide; and/or, optionally wherein the vector is a viral vector; and, optionally wherein the viral vector is a retroviral vector or a lentiviral vector.
9. An isolated host cell, comprising the chimeric receptor of any one of paragraphs 1-3; optionally wherein the host cell is a T lymphocyte or an NK cell; and, optionally wherein the T lymphocyte or the NK cell is activated and/or expanded *ex vivo.*
10. The host cell of paragraph 9, wherein the T lymphocyte is activated in the presence of one or more agents selected from the group consisting of anti-CD3/CD28, IL-2, and phytohemoagglutinin, or wherein the NK cell is activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line; and/or, optionally wherein the host cell is an autologous T lymphocyte or an autologous NK cell isolated from a patient having a cancer, or an allogeneic T lymphocyte or an allogeneic NK cell; and, optionally wherein the endogenous T cell receptor in the allogeneic T lymphocyte has been inhibited or eliminated.
11. A pharmaceutical composition, comprising the vector of paragraph 8 or a host cell of any one of paragraphs 9 or 10, and a pharmaceutically acceptable carrier or excipient; optionally wherein the pharmaceutical composition further comprises an antibody which exerts cytotoxicity to cancer cells; and, optionally wherein the antibody binds to cancer cells and has a human or humanized Fc portion that binds to human CD16, or wherein the antibody is selected from the group consisting of Rituximab, Trastuzumab, hu14.18K322A, Epratuzumab, Cetuximab, and Labetuzumab.
12. A T lymphocyte or NK cell expressing the chimeric receptor of any one of paragraphs 1-3 for use in enhancing the efficacy of an antibody-based immunotherapy of a cancer in a subject in need thereof, wherein the subject is being treated with an antibody which binds to cancer cells, and wherein the subject is optionally administered with an effective amount of interleukin-2 (IL-2).
13. A T lymphocyte or NK cell expressing the chimeric receptor of any one of paragraphs 1-3 for use in enhancing a T lymphocyte or an NK cell antibody-dependent cell cytotoxicity (ADCC) in a subject; optionally wherein the subject is being treated with an antibody which binds to cancer cells; and/or, optionally wherein the subject is a human patient having a cancer, and/or optionally wherein the subject is administered an effective amount of interleukine-2 (IL-2).
14. The T lymphocyte or NK cell for use according to paragraph 12 or paragraph 13, wherein the antibody has a human or humanized Fc portion, which binds to human CD16, or wherein the antibody is selected from the group consisting of Rituximab, Trastuzumab, hu14.18K322A, Epratuzumab, Cetuximab, and Labetuzumab; and/or, optionally wherein the cancer is selected from the group consisting of carcinoma, lymphoma, sarcoma, blastoma, and leukemia, or wherein the cancer is selected from the group consisting of a cancer of B-cell origin, breast cancer, gastric cancer, neuroblastoma, osteosarcoma, lung cancer, melanoma, prostate cancer, colon cancer, renal cell carcinoma, ovarian cancer, rhabdomyosarcoma, leukemia, and Hodgkin's lymphoma; and, optionally wherein the cancer of B-cell origin is selected from the group consisting of B-lineage acute lymphoblastic leukemia, B-cell chronic lymphocytic leukemia, and B-cell non-Hodgkin's lymphoma.
15. The T lymphocyte or NK cell for use according to any of paragraphs 12 to 14, wherein the T lymphocytes or NK cells are autologous T lymphocytes or autologous NK cells isolated from the subject, the autologous T lymphocytes or NK cells being optionally activated and/or expanded *ex vivo* prior to administering to the subject; or wherein the T lymphocytes or NK cells are allogeneic T lymphocytes, in which the expression of the endogenous T cell receptor has been optionally inhibited or eliminated, or allogeneic NK cells; the allogenic T lymphocytes or NK cells being optionally activated and/or expanded *ex vivo* prior to administering to the subject.
16. The T lymphocyte or NK cell for use according to any of paragraphs 12 to 15, wherein the chimeric receptor is introduced into the T lymphocytes or the NK cells by a method selected from the group consisting of retroviral transduction, lentiviral transduction, DNA electroporation, and RNA electroporation.
17. The T lymphocyte or NK cell for use according to paragraph 15 or paragraph 16, wherein the T lymphocyte is activated in the presence of one or more agents selected from the group consisting of anti-CD3/CD28, IL-2, and phytohaemoagglutinin, or wherein the NK cell is activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line.

The present disclosure is based, at least in part, on the development of novel chimeric receptors for use in, *e.g.,* cancer therapy. Accordingly, provided herein are chimeric receptors, nucleic acids encoding such, vectors comprising the encoding nucleic acids, host cells expressing the chimeric receptors, and uses of such host cells for enhancing antibody-based cancer therapy and/or ADCC effects in a subject, *e.g.,* a cancer patient.

In one aspect, the present disclosure provides a chimeric receptor comprising: (a) an extracellular ligand-binding domain of a CD 16 molecule; (b) the hinge and transmembrane domains of CD8α; and (c) a cytoplasmic domain comprising a 4-1BB signaling domain and a CD3ζ signaling domain. In some options, the CD16 molecule is F158 *FCGR3A* and its extracellular ligand-binding domain may comprise (*e.g.,* consists of) the amino acid sequence of SEQ ID NO: 16. In other options, the CD16 molecule is the V158 *FCGR3A* variant and its extracellular ligand-binding domain may comprise (*e.g.,* consists of) the amino acid sequence of SEQ ID NO: 2.

In any of the chimeric receptors disclosed herein, the hinge and transmembrane domains of CD8α may comprise (*e.g.,* consists of) the amino acid sequence of SEQ ID NO: 3; the 4-1BB signaling domain may comprise (*e.g.,* consists of) the amino acid sequence of SEQ ID NO: 4; and/or the CD3ζ signaling domain may comprise (*e.g.,* consists of) the amino acid sequence of SEQ ID NO: 5.

Any of the chimeric receptors disclosed herein may further comprise a signal peptide of CD8α, which is located at the N-terminus of the chimeric receptor. In one example, such signal peptide of CD8α may comprise (*e.g.,* consists of) the amino acid sequence of SEQ ID NO: 6.

In some examples, the chimeric receptor may comprise the amino acid sequence of residues 22-436 of SEQ ID NO: 1 or residues 22-436 of SEQ ID NO: 15. In one example, the the chimeric receptor comprise (*e.g.,* consists of) the amino acid sequence or SEQ ID NO: 1 or SEQ ID NO:15.

In another aspect, the present disclosure provides an isolated polynucleotide comprising a nucleotide sequence that encodes any of the chimeric receptors disclosed herein. In some examples, the polynucleotide encoding the chimeric receptor may comprise the nucleotide sequence of SEQ ID NO:10 or SEQ ID NO:18, the nucleotide sequence of SEQ ID NO: 11, the nucleotide sequence of SEQ ID NO: 12, the nucleotide sequence of SEQ ID NO: 14, or a combination thereof. In one specific option, the polynucleotide encoding the chimeric receptor comprises the nucleotide sequence of SEQ ID NO: 9 or SEQ ID NO: 17.

In a further aspect, the present disclosure provides a vector comprising a polynucleotide encoding any of the chimeric receptors disclosed herein, wherein the polynucleotide may be operatively linked to at least one regulatory element for expression of the chimeric receptor. In one examples, the vector is a viral vector (*e.g.,* a retroviral vector or a lentiviral vector).

Further, the present disclosure provides an isolated host cell comprising any of the chimeric receptors disclosed herein. In one specific option, the host cell is a T lymphocyte or an NK cell. In one specific option, the host cell is a T lymphocyte or an NK cell which is activated and/or expanded *ex vivo* (*e.g.*, T lymphocyte can be activated in the presence of one or more agents selected from the group consisting of anti-CD3/CD28, IL-2, and phytohemoagglutinin; *e.g.,* NK cell can be activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line). In one specific option, the host cell is an autologous T lymphocyte or an autologous NK cell isolated from a patient having a cancer. In one specific option, the host cell is an allogeneic T lymphocyte or an allogeneic NK cell. In one specific option, the host cell is an allogeneic T lymphocyte in which the expression of the endogenous T cell receptor has been inhibited or eliminated.

In yet another aspect, the present disclosure provides a pharmaceutical composition comprising (i) a polynucleotide encoding any of the chimeric receptors disclosed herein or a vector comprising such a polynucleotide, or a host cell expressing the chimeric receptor; and (ii) a pharmaceutically acceptable carrier or excipient. The pharmaceutical composition may further comprise an antibody, which exerts cytotoxicity to cancer cells (*e.g.,* an antibody which binds to cancer cells and has a human or humanized Fc portion which binds to human CD16, or Rituximab, or Trastuzumab, or hu14.18K322A, or Epratuzumab, or Cetuximab, or Labetuzumab).

In still another aspect, the present disclosure provides a method for enhancing efficacy of an antibody-based immunotherapy of a cancer in a subject in need thereof. The subject may be treated with an antibody which binds to cancer cells. Such a method may comprise introducing into the subject a therapeutically effective amount of T lymphocytes or NK cells, which express any of the chimeric receptors as described herein.

Moreover, the present disclosure provides a method of enhancing a T lymphocyte or an NK cell antibody-dependent cell cytotoxicity (ADCC) in a subject, the method comprising introducing into the subject a therapeutically effective amount of T lymphocytes or NK cells, which express the chimeric receptor as disclosed herein. In one specific option, the subject is being treated with an antibody which can bind to cancer cells.

In any of the methods described herein, the subject may be treated with an antibody which may have a human or humanized Fc portion which can bind to human CD16. In one specific option, the subject is being treated with an antibody selected from the group consisting of Rituximab, Trastuzumab, hu14.18K322A, Epratuzumab, Cetuximab, and Labetuzumab. In one specific option, the cancer is selected from the group consisting of carcinomas, lymphomas, sarcomas, blastomas, and leukemias. In one specific option, the cancer is selected from the group consisting of a cancer of B-cell origin (*e.g.,* B-lineage acute lymphoblastic leukemia, B-cell chronic lymphocytic leukemia, B-cell non-Hodgkin's lymphoma), breast cancer, gastric cancer, neuroblastoma, osteosarcoma, lung cancer, melanoma, prostate cancer, colon cancer, renal cell carcinoma, ovarian cancer, rhabdomyosarcoma, leukemia, and Hodgkin's lymphoma. In one specific option, the T lymphocytes or NK cells are autologous T lymphocytes or autologous NK cells isolated from the subject. In one specific option, prior to re-introduction into the subject, the autologous T lymphocytes or autologous NK cells are activated and/or expanded *ex vivo.* In one specific option, the T lymphocytes or NK cells are allogeneic T lymphocytes or allogeneic NK cells. In one specific option, the allogeneic T lymphocytes are T lymphocytes in which the expression of the endogenous T cell receptor has been inhibited or eliminated. In one specific option, prior to introduction into the subject, the allogeneic T lymphocytes or allogeneic NK cells are activated and/or expanded *ex vivo.* In one specific option, the chimeric receptor is introduced into the T lymphocytes or the NK cells by a method selected from the group consisting of retroviral transduction, lentiviral transduction, DNA electroporation, and RNA electroporation.

In any of the methods disclosed herein that involves T lymphocyte activation, the T lymphocytes can be activated in the presence of one or more agents selected from the group consisting of anti-CD3/CD28, IL-2, and phytohemoagglutinin. In any of the methods disclosed herein that involves NK cell activation, NK cells can be activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line.

Any of the methods disclosed herein may further comprise administering to the subject a therapeutically effective amount of IL-2.

Also within the scope of the present disclosure are (i) pharmaceutical compositions for use for enhancing ADCC effect in cancer patients or for treating cancer, the pharmaceutical composition comprises any of the polynucleotides disclosed herein that encode a chimeric receptor also disclosed herein, a vector comprising such a polynucleotide, or host cells expressing the chimeric receptor, and a pharmaceutically acceptable carrier or excipient; and (ii) uses of such pharmaceutical compositions for the manufacture of a medicament for the treatment of disease cancer. The pharmaceutical compositions may further comprise an anti-cancer antibody, such as those known in the art or disclosed herein.

The details of one or more options of the disclosure are set forth in the description below. Other features or advantages of the present disclosure will be apparent from the following drawings and detailed description of severaloptions, and also from the appended claims.

### BRIEF DESCRIPTION OF THE DRAWINGS

This patent application file contains at least one drawing executed in color. Copies of this patent application with color drawing(s) will be provided by the Office upon request and payment of the necessary fee.

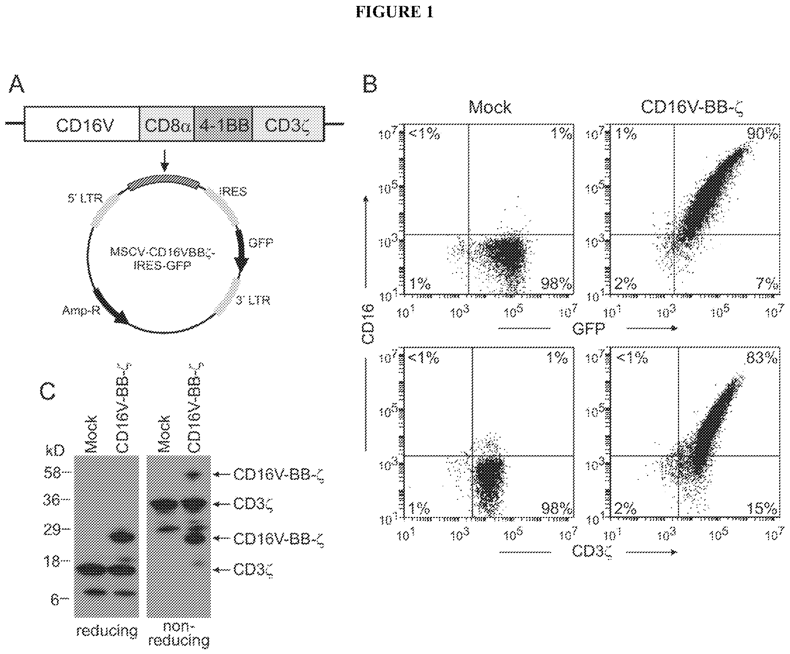

**Figures 1** demonstrates expression of CD16V-BB-ζ receptors in T cells. **A.** Schematic representation of the CD16V-BB-ζ receptor construct. **B.** Expression of CD16V-BB-ζ receptors in peripheral blood T lymphocytes. Flow cytometric dot plots illustrate expression of CD 16 (B73.1 antibody) in combination with GFP or CD3ζ in activated T lymphocytes transduced with a vector containing GFP alone (Mock) or GFP and CD16V-BB-ζ. Percentage of positive cells in each quadrant is shown. **C.** Western blotting of cell lysates from T lymphocytes transduced with GFP alone or CD16V-BB-ζ. The membranes were probed with an anti-CD3ζ antibody.

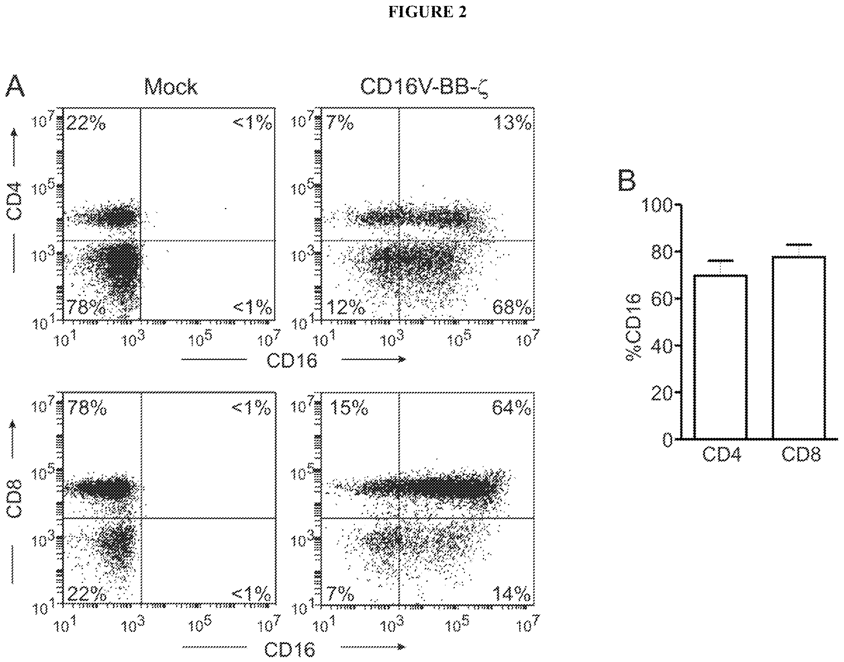

**Figures 2** shows expression of CD16V-BB-ζ receptor in T-cell subsets. **A.** Activated CD3+ T lymphocytes were transduced with a vector containing GFP alone (Mock) and with a vector containing the CD16V-BB-ζ construct. Expression of CD16 was tested in CD4+ and CD8+ cells by flow cytometry. Dot plots show results of one representative experiment. **B.** Summary of results (mean ± SD) obtained with T lymphocytes from 3 donors (P = N.S.).

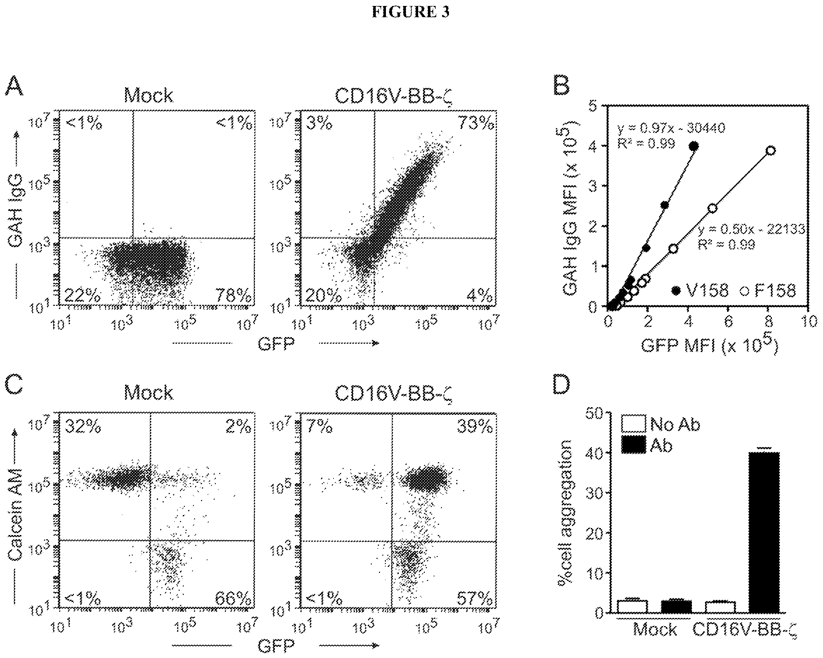

**Figures 3** demonstrates antibody-binding capacity of CD16V-BB-ζ receptors. **A.** T lymphocytes transduced with a vector containing GFP (Mock) or GFP and CD16V-BB-ζ were incubated with Rituximab for 30 minutes; the amount of antibody bound to the cell surface was visualized with a goat-anti human IgG antibody conjugated to phycoerythrin (GAH IgG) and flow cytometry. **B.** Jurkat cells transduced with CD16V-BB-ζ (V158) or CD16F-BB-ζ (F158) were incubated with Rituximab for 30 minutes. The plot compares the relation between mean fluorescence intensity (MFI) of GFP and MFI of GAH IgG obtained with cells expressing the two receptors. **C.** Jurkat cells mock-transduced or transduced with CD16V-BB-ζ were co-cultured with Daudi cells labeled with calcein AM orange-red in the presence of Rituximab. Cell aggregates are quantified in the upper right quadrants of each dot plot. **D.** Summary of the aggregation assays illustrated in C. Bars show mean ± SD of 3 experiments. Aggregation measured with Jurkat cells transduced with CD16V-BB-ζ in the presence of Rituximab ("Ab") was significantly higher than that measured in the 3 other culture conditions (P<0.001 by *t* test).

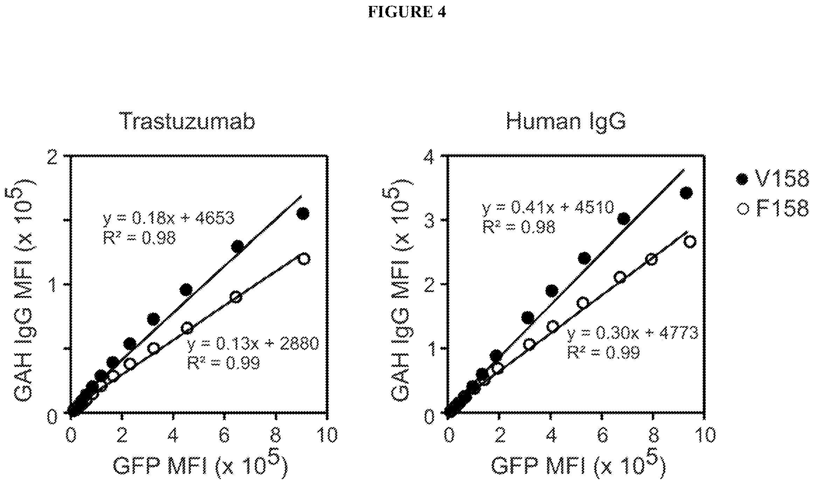

**Figure 4** shows the relative capacity of CD16V-BB-ζ and CD16F-BB-ζ receptors to bind Trastuzumab and human IgG. Jurkat cells transduced with CD16V-BB-ζ (V158; black symbols) or CD16F-BB-ζ (F158; white symbols) were incubated with Trastuzumab or human IgG for 30 minutes. The plots compare the relation between mean fluorescence intensity (MFI) of GFP and MFI of goat-anti human (GAH) IgG conjugated to PE obtained with cells expressing either receptor (P <0.0001 for both Trastuzumab and IgG).

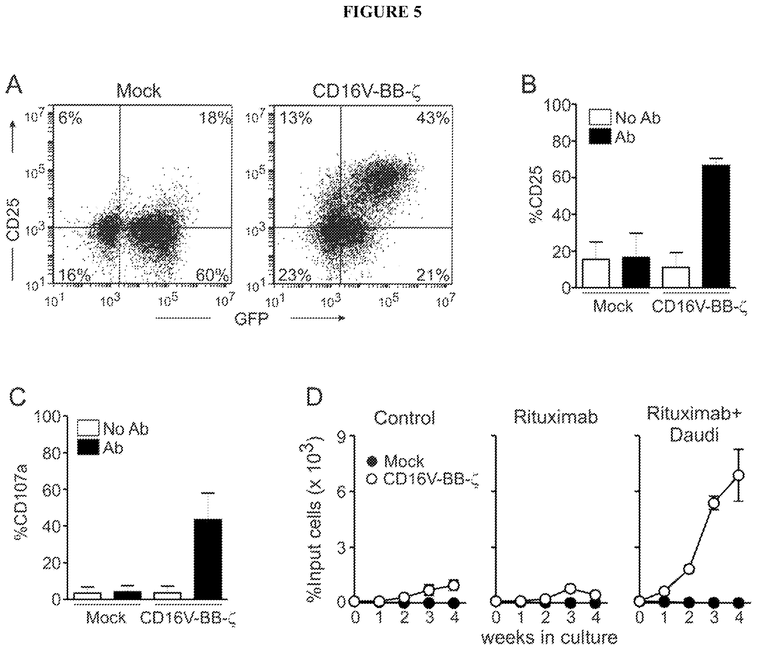

**Figures 5** demonstrates that immunoglobulin binding to CD16V-BB-ζ receptors induces T cell activation, exocytosis of lytic granules, and cell proliferation. **A.** T lymphocytes transduced with a vector containing GFP (Mock) or GFP and CD16V-BB-ζ were cultured in microtiter plates coated with Rituximab for 48 hours without IL-2; expression of CD25 was measured by flow cytometry. **B.** Summary of the results of the test illustrated in A: bars show CD25 expression in GFP+ cells (mean ± SD of experiments with T cells from 3 donors); CD25 expression was significantly higher in T lymphocytes transduced with CD16V-BB-ζ in the presence of Rituximab ("Ab") than in the other experimental conditions (P ≤0.003). C. T lymphocytes from 4 donors were transduced with a vector containing GFP (Mock) or GFP and CD16V-BB-ζ were cultured as in A (n= 3) or with Daudi cells (n =3) for 4 hours; CD107a staining was measured by flow cytometry. Bars show mean ± SD of the 6 experiments; CD107a expression was significantly higher in T lymphocytes transduced with CD16V-BB-ζ in the presence of Rituximab ("Ab") than in the other experimental conditions (P <0.0001). **D.** Mock- or CD16V-BB-ζ-transduced T lymphocytes were cultured alone, or with Rituximab with or without Daudi cells for up to 4 weeks. Symbols indicate percentage of cell recovery as compared to the number of input cells (mean ± SD of experiments with T cells from 3 donors).

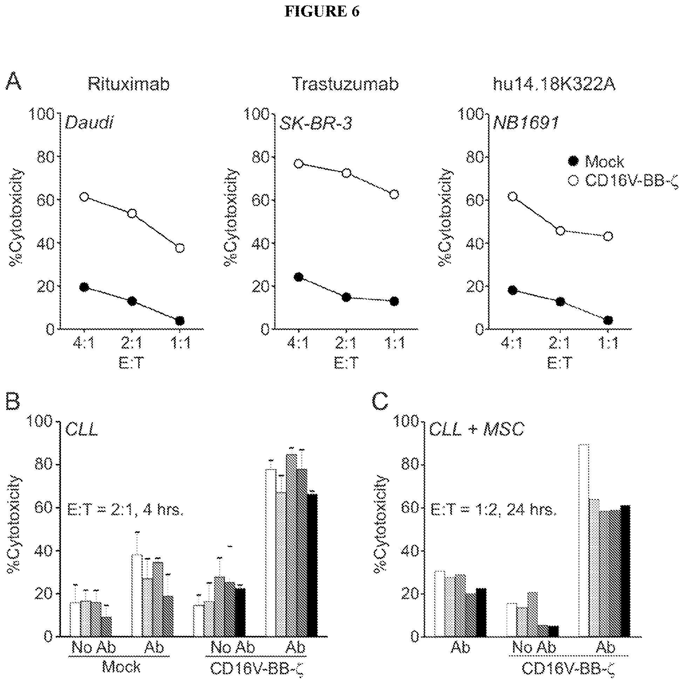

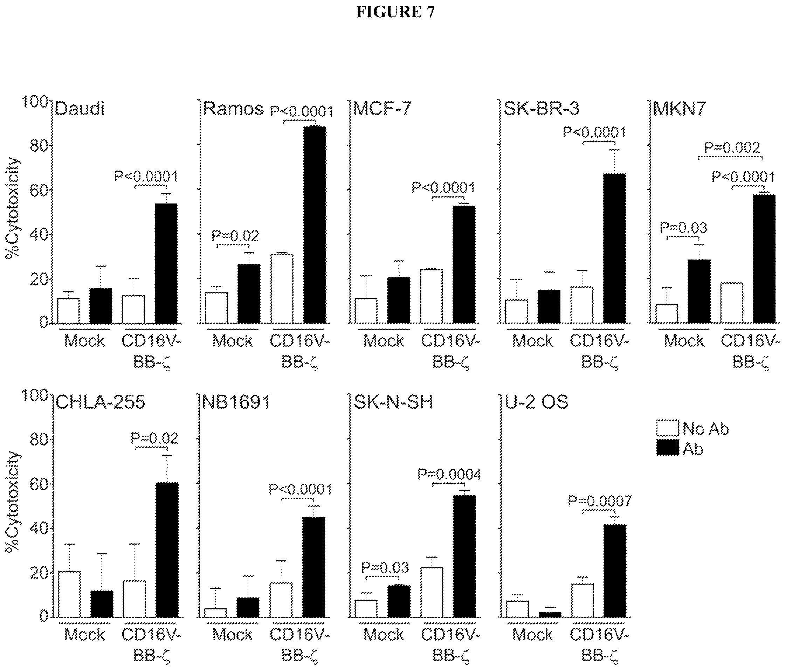

**Figures 6** demonstrates antibody-dependent cell cytotoxicity mediated by CD16V-BB-ζ T lymphocytes *in vitro.* **A.** Representative examples of cytotoxicity against cancer cell lines mediated by mock- or CD16V-BB-ζ-transduced T lymphocytes in the presence of the corresponding antibody. Each symbol indicates the mean of triplicate cultures (P<0.01 by paired *t* test for all 3 comparisons). The full set of data is shown in Fig. 7. **B.** Cytotoxicity of mock- or CD16V-BB-ζ-transduced T lymphocytes, with or without Rituximab ("Ab"), against primary cells from patients with chronic lymphocytic leukemia (CLL). Each bar (with a different shade for each patient) corresponds to mean (± SD) cytotoxicity as determined in triplicate 4-hour assays at 2:1 E:T ratio. Cytotoxicity with CD16V-BB-ζ T cells and antibody was significantly higher than that measured in any of the other 3 conditions (P <0.0001 by *t* test); with mock-transduced T cells, the addition of antibody increased cytotoxicity (P = 0.016); all other comparisons: P >0.05. C. Cytotoxicity against the same CLL samples tested in B after 24 hours at 1:2 E:T in the presence of mesenchymal stromal cells (MSC). Each bar corresponds to the average of two tests. Cytotoxicity with CD16V-BB-ζ T cells plus antibody was significantly higher than that with antibody alone (P = 0.0002) or cells alone (P <0.0001); cytotoxicity with antibody alone was significantly higher than that with cells alone (P = 0.0045).
**Figure 7** shows the collective results of 4-hour in vitro cytotoxicity assays. Mock- or CD16V-BB-ζ T lymphocytes were cocultured with the cell lines shown and either non-reactive human immunoglobulin ("No Ab") or the corresponding antibody ("Ab"). This was Rituximab for Daudi and Ramos, Trastuzumab for MCF-7, SKBR-3, and MKN-7, and hu14.18K322A for CHLA-255, NB1691, SK-N-SH and U-2 OS. Shown are cytotoxicities at 2:1 ratio (4:1 for CHLA-255) as compared to tumor cells cultured without T cells and/or antibody. Results correspond to mean (± SD) cytotoxicity of triplicate experiments performed with T lymphocytes of 3 donors for NB1691 and SK-BR-3, and of 1 donor for the remaining cell lines; results of Daudi are mean (± SD) cytotoxicity of triplicate measurements from 2 donors and single measurements with T lymphocytes from 4 additional donors. Mean cytotoxicity of Rituximab, Trastuzumab or hu14.18K322A when added to cultures in the absence of T cells was <10%.

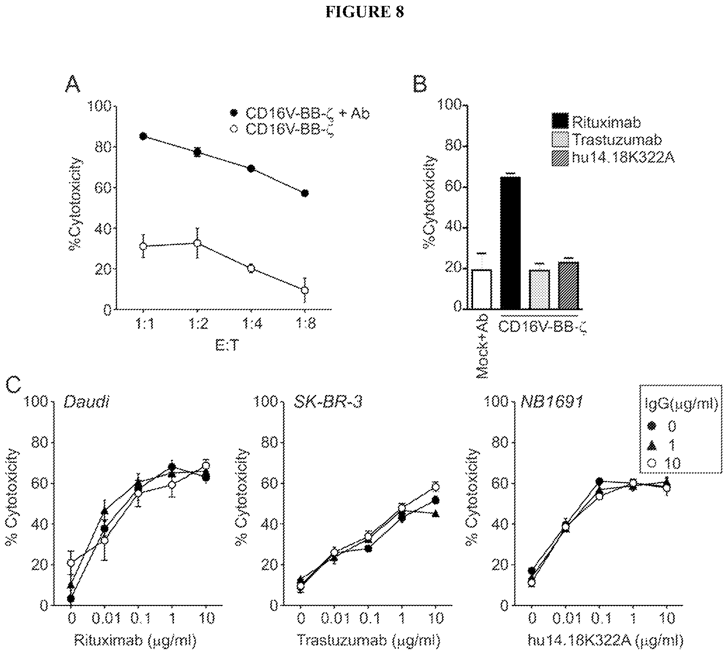

**Figures 8** demonstrates that **c**ytotoxicity of CD16V-BB-ζ T lymphocytes is powerful, specific and is not affected by unbound IgG. **A.** CD16V-BB-ζ T lymphocytes were cocultured with the neuroblastoma cell line NB1691 with either non-reactive human immunoglobulin ("No Ab") or the hu14.18K322A antibody ("Ab") for 24 hours. Results correspond to mean (± SD) cytotoxicity of triplicate experiments. Cytotoxicity remained significantly higher with CD16V-BB-ζ cells plus hu14.18K322A antibody as compared to CD16V-BB-ζ T cells alone even at 1:8 E:T (P = 0.0002). **B.** Mock- or CD16V-BB-ζ -transduced T lymphocytes were cocultured with the B-cell lymphoma cell line Daudi for 4 hours at 2:1 E:T in the presence of Rituximab or the non-reactive antibodies Trastuzumab or hu14.18K322A. Results correspond to mean (± SD) cytotoxicity of triplicate experiments ("Mock" results are the aggregate of triplicate experiments with each antibody). Cytotoxicity with Rituximab was significantly higher than those in all other experimental conditions (P <0.0001 for all comparisons). **C.** Cytotoxicity of T lymphocytes expressing CD16V-BB-ζ against tumor cell lines at 8 : 1 E : T in the presence of various concentrations of immunotherapeutic antibodies and competing unbound IgG (added simultaneously to the antibody). Symbols correspond to mean (± SD) of at least triplicate measurement for each antibody concentration. For each cell line, cytotoxicities were not statistically different, regardless of the amount of unbound IgG present.

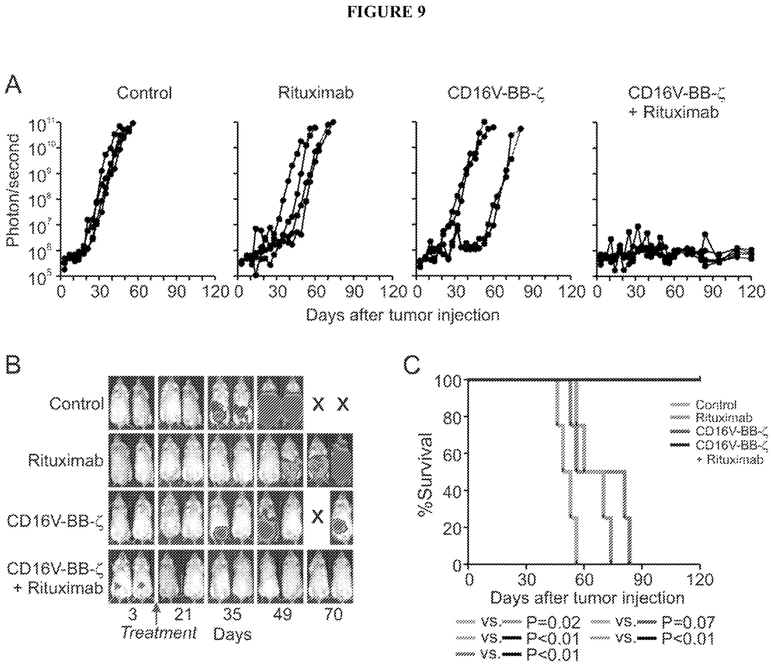

**Figures 9** demonstrates that T lymphocytes expressing CD16V-BB-ζ receptors exert anti-tumor activity in vivo. NOD-SCID-IL2RGnull mice were injected i.p. with 3 x 10⁵ Daudi cells labeled with luciferase. Rituximab (150 µg) was injected i.p. once weekly for 4 weeks starting on day 4. In 4 mice, no other treatment was given, while in 5 other mice, the first Rituximab injection was followed by T lymphocytes expressing CD16V-BB-ζ receptors (1 x 10⁷ i.p.; n = 5) on days 5 and 6; other 2 groups of 4 mice each received CD16V-BB-ζ T lymphocytes preceded by *i.p.* injection of RPMI-1640 instead of Rituximab, or i.p. injection of RPMI-1640 medium only ("Control"). **A.** Results of in vivo imaging of tumor growth. Each symbol corresponds to one bioluminescence measurement; lines connect all measurements in one mouse. **B.** Representative mice (2 per group) for each experimental condition. Ventral images on day 3 were processed with enhanced sensitivity to demonstrate the presence of tumors in mice of the CD16V-BB-ζ + Rituximab group. Mice were euthanized when bioluminescence reached 5 x 10¹⁰ photons/second. **C.** Overall survival comparisons of mice in the different treatment groups.

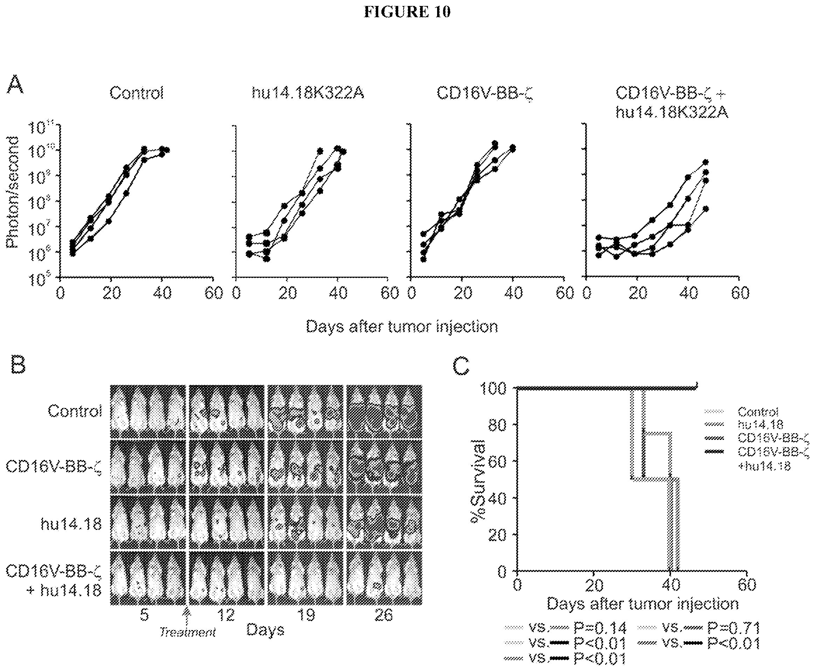

**Figures 10** confirms that T lymphocytes expressing CD16V-BB-ζ receptors exert anti-tumor activity in vivo. NOD-SCID-IL2RGnull mice were injected i.p. with 3 x 10⁵ NB1691 cells labeled with luciferase. Hu14.18K322A antibody (25 µg) was injected i.p. once weekly for 4 weeks starting on day 5. In 4 mice, no other treatment was given, while in 4 other mice, the first antibody injection was followed by T lymphocytes expressing CD16V-BB-ζ receptors (1 x 10⁷ i.p.; n = 4) on days 6 and 7; other 2 groups of 4 mice each received CD16V-BB-ζ T lymphocytes preceded by *i.p.* injection of RPMI-1640 instead of antibody, or *i.p.* injection of RPMI-1640 medium only ("Control"). **A.** Results of in vivo imaging of tumor growth. Each symbol corresponds to one bioluminescence measurement; lines connect all measurements in one mouse. **B.** Images of all mice for each experimental condition. Mice were euthanized when bioluminescence reached 1 x 10¹⁰ photons/second. **C.** Overall survival comparisons of mice in the different treatment groups.

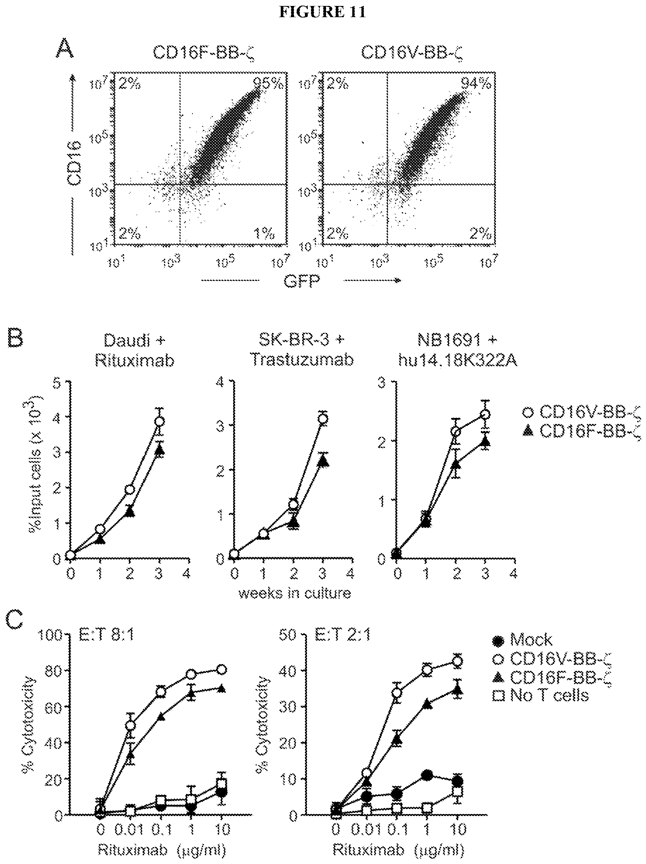

**Figures 11** demonstrates functional differences between T lymphocytes expressing CD16V-BB-ζ and CD16F-BB-ζ receptors. **A.** Flow cytometric dot plots show expression of CD16 (detected with the B73.1 antibody) and green fluorescent protein (GFP) in T lymphocytes transduced with CD16V-BB-ζ or CD16F-BB-ζ. Percentage of positive cells in each quadrant is shown. **B.** T lymphocytes transduced with either CD16V or CD16F receptor were cultured with Daudi, SK-BR-3 or NB1691 cells in the presence of Rituximab, Trastuzumab and hu14.18K322A, respectively. All antibodies were used at 0.1 µg/mL. Symbols indicate percentage of cell recovery as compared to the number of input cells (mean ± SD of 3 experiments); cell counts for weeks 1-3 of culture were significantly different by paired *t* test for all 3 cultures (Daudi, P = 0.0007; SK-BR-3, P = 0.0164; NB1691, P = 0.022). C. Antibody-dependent cell cytotoxicity mediated by T lymphocytes expressing either CD16V-BB-ζ or CD16F-BB-ζ receptors against Daudi cells in the presence of various concentrations of Rituximab. Each symbol indicates the mean ± SD of triplicate cultures at 8:1 (left) or 2:1 (right) E:T. Cytotoxicities of T cells with CD16V-BB-ζ were significantly higher than those of T cells with CD16F-BB-ζ (P <0.001 for either E:T).

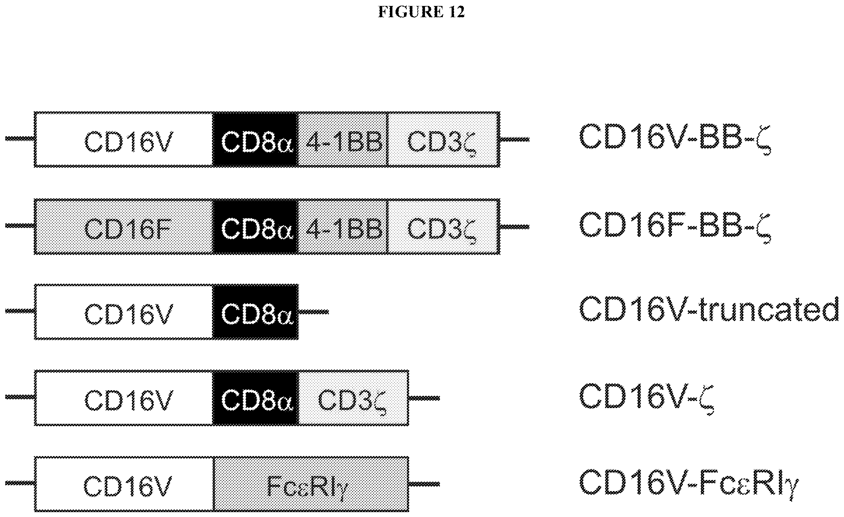

**Figure 12** shows a schematic representation of CD16 chimeric receptors used in this study.

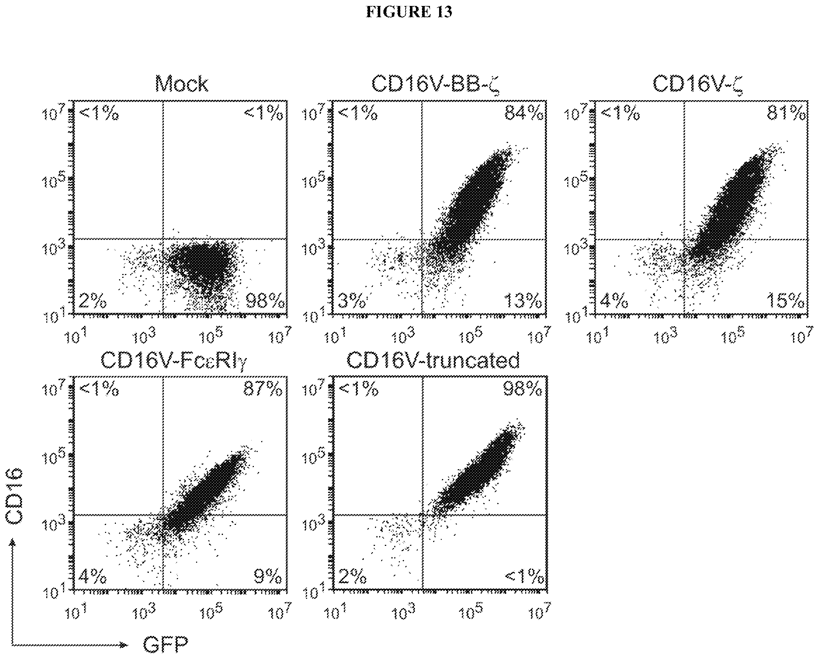

**Figure 13** shows expression of CD16V receptors with different signaling domains. Flow cytometric dot plots illustrate expression of CD 16 (detected with the 3G8 antibody) in combination with GFP in activated T lymphocytes transduced with a vector containing green fluorescent protein (GFP) alone (Mock) or different CD16V constructs. Percentage of positive cells in each quadrant is shown.

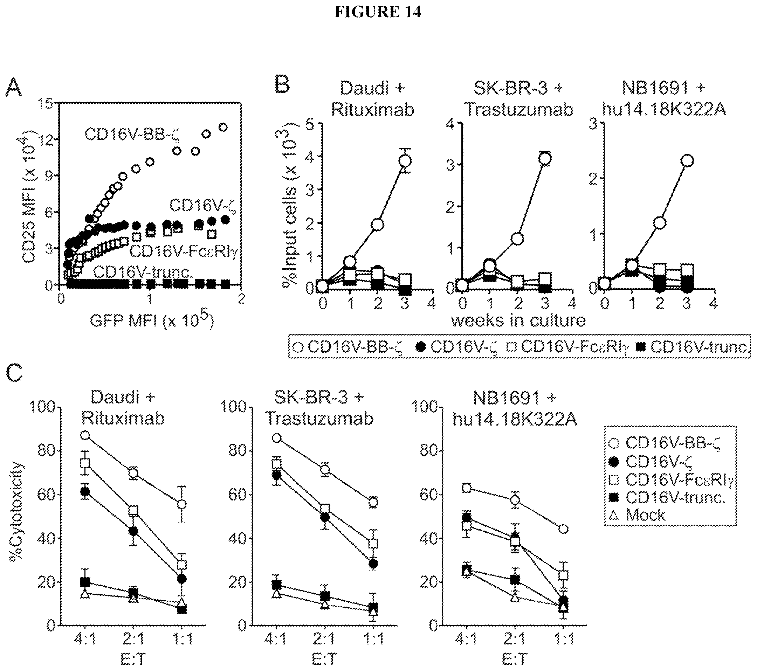

**Figures 14** demonstrates that CD16V-BB-ζ induces higher T cell activation, proliferation and cytotoxicity than CD16V receptors with different signaling properties. **A.** CD25 mean fluorescence intensity (MFI) by flow cytometry plotted against green fluorescent protein (GFP) MFI in T lymphocytes expressing different chimeric receptors after 48-hour co-culture with Daudi cells and Rituximab (0.1 µg/mL). CD25 expression with CD16V-BB-ζ was significantly higher than that triggered by CD16V-ζ, CD16V-FcεRIγ or CD16V with no signaling capacity ("CD16V-trunc.") (P<0.0001 by linear regression analysis). **B.** T lymphocytes transduced with various CD16V receptors were cultured with Daudi, SK-BR-3 or NB1691 cells in the presence of Rituximab, Trastuzumab and hu14.18K322A, respectively. All antibodies were used at 0.1 µg/mL. Symbols indicate percentage of cell recovery as compared to number of input cells (mean ± SD of 3 experiments); cell counts for weeks 1-3 of culture were significantly higher with CD16V-BB-ζ receptors that with all other receptors by paired *t* test for all 3 cultures (P<0.0001). C. ADCC of T lymphocytes expressing various CD16V receptors or mock-transduced T cells against Daudi, SK-BR-3 and NB1691 in the presence of Rituximab, Trastuzumab and hu14.18K322A, respectively. Symbols are mean ± SD of triplicate cultures at the E:T shown. Cytotoxicities with CD16V-BB-ζ receptors were significantly higher than those with all other receptors (P<0.0001 by *t* test in all comparisons) while cytotoxicities of lymphocytes mock-transduced or transduced with the CD16V-truncated receptor were not significantly different (P>0.05) from each other; cytotoxicity with CD16V-FcεRIγ was significantly higher than that with CD3-ζ against Daudi (P = 0.006) and SK-BR-3 (P = 0.019); lymphocytes expressing either receptor had higher cytotoxicities than those mock-transduced or transduced with CD16V-truncated (P<0.01 for all comparisons).

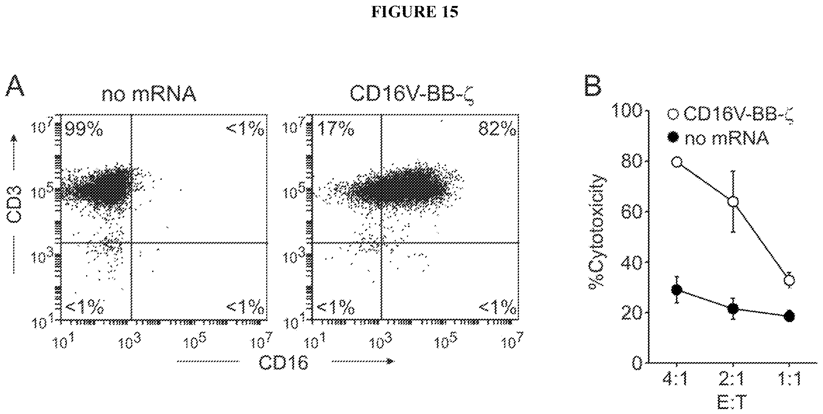

**Figures 15** demonstrates expression of CD16V-BB-ζ receptors by mRNA electroporation. **A.** Activated T lymphocytes were electroporated with CD16V-BB-ζ mRNA or without mRNA (Mock); expression of CD 16 was tested 24 hours later by flow cytometry. **B.** Cytotoxicity of mock or CD16V-BB-ζ electroporated T cells was tested against the Ramos cell line in the presence of Rituximab. Symbols show mean ± SD percent cytotoxicity (n =3; P <0.01 for comparisons at all E:T ratios).

### DETAILED DESCRIPTION OF THE PRESENT DISCLOSURE

The present disclosure is based on the construction of novel chimeric receptors, which enhances anti-cancer antibody efficacy in cancer treatment. T lymphocytes expressing αβ T-cell receptors (the vast majority of T cells) lack activating FcγR and do not mediate antibody-dependent cell cytotoxicity (ADCC). Nimmerjahn et al., Nat Rev Immunol. 2008;8(1):34-47. The present disclosure shows that expression of a chimeric receptor composed of a FcγR and T-cell signaling molecules should confer ADCC capability to these cells and, therefore, should significantly augment the anti-tumor potential of monoclonal antibodies (as well as other anti-tumor molecules comprising the Fc portion, such as, *e.g.,* a composite molecule constituted by a ligand (*e.g*., cytokine, immune cell receptor) binding a tumor surface receptor combined with the Fc-portion of an immunoglobulin or Fc-containing DNA or RNA), regardless of the targeted tumor-antigen. Described herein is the antibody-guided anti-tumor activity of T lymphocytes expressing such chimeric receptor.

The present disclosure provides a chimeric receptor which comprises (i) an extracellular ligand-binding domain of a CD16 molecule, which can be F158 *FCGR3A* or the high-affinity V158 *FCGR3A* variant, (ii) a hinge and transmembrane domains of CD8α, and (ii) the signaling domains of CD3ζ and 4-1BB. The chimeric receptor of the present disclosure is a universal chimeric receptor with potential for augmenting significantly the efficacy of antibody therapy against multiple tumors. As discussed in the Examples section, below, when expressed in human T cells by retroviral transduction, the receptor of the present disclosure has a significantly higher affinity for human IgG including humanized antibodies such as the anti-CD20 antibody Rituximab as compared to another receptor containing the common F158 variant. Engagement of the chimeric receptor provokes T-cell activation, exocytosis of lytic granules and proliferation. CD16V-BB-ζ expressing T cells specifically kill lymphoma cell lines and primary chronic lymphocytic leukemia (CLL) cells in the presence of Rituximab at low effector: target ratio, even when CLL cultures are performed on bone marrow-derived mesenchymal cells. The anti-HER2 antibody Trastuzumab trigger chimeric receptor-mediated antibody-dependent cell cytotoxicity (ADCC) against breast and gastric cancer cells, and the anti-GD2 antibody hu14.18K322A against neuroblastoma and osteosarcoma cells. As further disclosed in the Examples section, T cells expressing the chimeric receptor and Rituximab in combination eradicated human lymphoma cells in immunodeficient mice, while T cells or antibody alone did not. To facilitate clinical translation of this technology, a method based on electroporation of the chimeric receptor mRNA was developed as disclosed herein, leading to efficient and transient receptor expression without the use of viral vectors.

### Definitions

The term "chimeric receptor" as used herein is defined as a cell-surface receptor comprising an extracellular ligand binding domain, a transmembrane domain and a cytoplasmic signaling domain in a combination that is not naturally found together on a single protein. The chimeric receptor of the present disclosure is intended primarily for use with T cells but could also be used for natural killer (NK) cells.

The term "about" or "approximately" means within an acceptable error range for the particular value as determined by one of ordinary skill in the art, which will depend in part on how the value is measured or determined, *i.e.,* the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1% of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

In the context of the present disclosure insofar as it relates to any of the disease conditions recited herein, the terms "treat", "treatment", and the like mean to relieve or alleviate at least one symptom associated with such condition, or to slow or reverse the progression of such condition. Within the meaning of the present disclosure, the term "treat" also denotes to arrest, delay the onset (*i.e.*, the period prior to clinical manifestation of a disease) and/or reduce the risk of developing or worsening a disease. E.g., in connection with cancer the term "treat" may mean eliminate or reduce a patient's tumor burden, or prevent, delay or inhibit metastasis, etc.

As used herein the term "therapeutically effective" applied to dose or amount refers to that quantity of a compound or pharmaceutical composition (*e.g*., a composition comprising T lymphocytes (and/or NK cells) comprising the chimeric receptor of the present disclosure, and optionally further comprising a tumor-specific cytotoxic monoclonal antibody or another anti-tumor molecule comprising the Fc portion (*e.g.,* a composite molecule constituted by a ligand (*e.g*., cytokine, immune cell receptor) binding a tumor surface receptor combined with the Fc-portion of an immunoglobulin or Fc-containing DNA or RNA)) that is sufficient to result in a desired activity upon administration to a subject in need thereof. Within the context of the present disclosure, the term "therapeutically effective" refers to that quantity of a compound or pharmaceutical composition that is sufficient to delay the manifestation, arrest the progression, relieve or alleviate at least one symptom of a disorder treated by the methods of the present disclosure. Note that when a combination of active ingredients is administered the effective amount of the combination may or may not include amounts of each ingredient that would have been effective if administered individually.

The phrase "pharmaceutically acceptable", as used in connection with compositions of the present disclosure, refers to molecular entities and other ingredients of such compositions that are physiologically tolerable and do not typically produce untoward reactions when administered to a mammal (*e.g*., a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

As used herein, the term "subject" refers to any mammal. In a preferred option, the subject is human.

As used in this specification and the appended claims, the singular forms "a," "an," and "the" include plural references unless the context clearly dictates otherwise.

In accordance with the present present disclosure there may be employed conventional molecular biology, microbiology, and recombinant DNA techniques within the skill of the art. Such techniques are explained fully in the literature. See, *e.g*., Sambrook, Fritsch & Maniatis, Molecular Cloning: A Laboratory Manual, Second Edition (1989) Cold Spring Harbor Laboratory Press, Cold Spring Harbor, New York (herein "Sambrook *et al.,* 1989"); DNA Cloning: A practical Approach, Volumes I and II (D.N. Glover ed. 1985); Oligonucleotide Synthesis (MJ. Gait ed. 1984); Nucleic Acid Hybridization (B.D. Hames & S.J. Higgins eds.(1985»; Transcription and Translation (B.D. Hames & S.J. Higgins, eds. (1984»; Animal Cell Culture (R.I. Freshney, ed. (1986»; Immobilized Cells and Enzymes (1RL Press, (1986»; B. Perbal, A practical Guide To Molecular Cloning (1984); F.M. Ausubel et al. (eds.), Current Protocols in Molecular Biology, John Wiley & Sons, Inc. (1994); among others.

### Chimeric Receptors of the Present disclosure

The present disclosure provides a chimeric receptor comprising: (a) an extracellular ligand-binding domain of F158 *FCGR3A* or V158 *FCGR3A* variant *(e.g.,* SEQ ID NO:16 or SEQ ID NO:2, respectively); (b) a hinge and transmembrane domains of CD8α *(e.g.,* SEQ ID NO: 3); and (c) a cytoplasmic domain comprising a 4-1BB signaling domain (*e.g.,* SEQ ID NO:4) and a CD3ζ signaling domain (*e.g.,* SEQ ID NO:5). The chimeric receptor may further comprise a signal peptide of CD8α, *e.g.,* SEQ ID NO:6. In one specific option, the chimeric receptor comprises the amino acid sequence SEQ ID NO: 1 or SEQ ID NO: 15. In one example, the chimeric receptor is CD16V-BB-ζ that consists of the amino acid sequence of SEQ ID NO: 1. In another example, the chimeric receptor is CD16F-BB-ζ that consists of the amino acid sequence of SEQ ID NO: 15.

In one option, the chimeric receptor of the present disclosure contains one or more signaling domains in addition to the two signaling domains described herein, *i.e*., CD3ζ and 4-1BB/CD137. In one specific option, several signaling domains are fused together for additive or synergistic effect. Non-limiting examples of useful additional signaling domains include part or all of one or more of TCR Zeta chain, CD28, OX40/CD134, 4-1BB/CD137, FcεRIγ, ICOS/CD278, ILRB/CD122, IL-2RG/CD132, and CD40.

The present disclosure also provides polynucleotides encoding the chimeric receptors disclosed above. In one specific option, the polynucleotide encoding an extracellular ligand-binding domain of V158 *FCGR3A* variant comprises the nucleotide sequence of SEQ ID NO: 10. Alternatively, the polynucleotide encoding the F158 *FCGR3A* extracellular domain comprises the nucleotide sequence of SEQ ID NO:18. In one specific option, the polynucleotide encoding the hinge and transmembrane domains of CD8α comprises the nucleotide sequence of SEQ ID NO: 11. In one specific option, the polynucleotide encoding the 4-1BB signaling domain comprises the nucleotide sequence of SEQ ID NO: 12. In one specific option, the polynucleotide encoding the CD3ζ signaling domain comprises the nucleotide sequence of SEQ ID NO: 13. In one specific option, the polynucleotide encoding the signal peptide of CD8α comprises the nucleotide sequence of SEQ ID NO: 14.

In one example, the polynucleotide encoding the chimeric receptor of CD 16V-BB-ζ and comprises the nucleotide sequence of SEQ ID NO: 9. In another example, the polynucleotide encoding the chimeric receptor of CD16F-BB-ζ and comprises the nucleotide sequence of SEQ ID NO: 17.

In conjunction with the polynucleotides, the present disclosure also provides vectors comprising such polynucleotides (including vectors in which such polynucleotides are operatively linked to at least one regulatory element for expression of the chimeric receptor). Non-limiting examples of useful vectors of the present disclosure include viral vectors such as, *e.g.,* retroviral vectors and lentiviral vectors.

In one specific option, such vectors also include a suicide gene. As used herein, the term "suicide gene" refers to a gene that causes the cell expressing the suicide gene to die. The suicide gene can be a gene that confers sensitivity to an agent, *e.g.,* a drug, upon the cell in which the gene is expressed, and causes the cell to die when the cell is contacted with or exposed to the agent. Suicide genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and include, for example, the Herpes Simplex Virus (HSV) thymidine kinase (TK) gene, cytosine daminase, purine nucleoside phosphorylase, nitroreductase and caspases such as caspase 8.

The present disclosure also provides host cells comprising the chimeric receptors disclosed above. Non-limiting examples of useful host cells include T lymphocytes and NK cells, which can be either autologous or allogeneic (with endogenous T-cell receptor either removed or retained). In one specific option, the host cell is an autologous T lymphocyte isolated from a patient having cancer. In one specific option, such autologous T lymphocyte is activated and expanded *ex vivo.*

The chimeric receptor of the present disclosure can be introduced into the host cell by any method known in the art. Non-limiting examples of particularly useful methods include retroviral transduction, lentiviral transduction, and DNA and mRNA electroporation. As demonstrated in the Examples below, mRNA electroporation, results in effective expression of the chimeric receptor of the present disclosure in T lymphocytes. Examples of references describing retroviral transduction include Anderson et al., U.S. Pat. No. 5,399,346; Mann et al., Cell 33:153 (1983); Temin et al., U.S. Pat. No. 4,650,764; Temin et al., U.S. Pat. No. 4,980,289; Markowitz et al., J. Virol. 62:1120 (1988); Temin et al., U.S. Pat. No. 5,124,263; International Patent Publication No. WO 95/07358, published Mar. 16, 1995, by Dougherty et al.; and Kuo et al., Blood 82:845 (1993). International Patent Publication No. WO 95/07358 describes high efficiency transduction of primary B lymphocytes. See also the Examples section, below, for examples of specific techniques for retroviral transduction and mRNA electroporation which can be used.

Host cell activation and expansion is usually used to allow integration of a viral vector into the genome and expression of the gene encoding the chimeric receptor of the present disclosure. However, if mRNA electroporation is used, no activation and expansion is required (although electroporation is more effective when performed on activated cells). As a result of viral transduction, host cells (T lymphocytes or NKT cells) express the chimeric receptor of the present disclosure for a long time potentially producing a stronger effect than upon mRNA electroporation when the receptor is expressed transiently (typically for 3-5 days). However, viral transduction is complex, expensive and difficult to implement, while mRNA electroporation is much simpler and more easily implementable. In addition, transient expression is useful if there is a potential toxicity and should be helpful in the initial phases of clinical testing for possible side effects.

### Pharmaceutical Compositions of the Present disclosure

A further aspect of the present disclosure provides pharmaceutical compositions. In one option, the present disclosure provides a pharmaceutical composition comprising (i) a polynucleotide encoding the chimeric receptor of the present disclosure or a vector comprising such polynucleotide and (ii) a pharmaceutically acceptable carrier or excipient.

In another option, the present disclosure provides a pharmaceutical composition comprising (i) a host cell comprising the chimeric receptor of the present disclosure and (ii) a pharmaceutically acceptable carrier or excipient. In one specific option, such pharmaceutical composition further comprises a monoclonal antibody which can exert cytotoxicity to cancer cells (*e.g.,* Rituximab, Trastuzumab, hu14.18K322A, etc.) or another anti-tumor molecule comprising the Fc portion (e.g., a composite molecule constituted by a ligand (*e.g.,* cytokine, immune cell receptor) binding a tumor surface receptor combined with the Fc-portion of an immunoglobulin or Fc-containing DNA or RNA).

Suitable excipients for use in the pharmaceutical compositions of the present disclosure will be well known to those of skill in the art and may, for example, comprise tissue culture medium (*e.g.,* for cells to survive *ex vivo*) or a saline solution (*e.g.,* when cells are being injected in patients). A thorough discussion of pharmaceutically acceptable excipients is available in Remington's Pharmaceutical Sciences (Mack Pub. Co., N.J. 1991).

The pharmaceutical compositions of the present disclosure may also contain one or more additional active compounds as necessary for the particular indication being treated, preferably those with complementary activities that do not adversely affect each other. Non-limiting examples of possible additional active compounds include, *e.g.,* IL2 as well as various agents listed in the discussion of combination treatments, below.

### Therapeutic Methods of the Present disclosure

The chimeric receptor of the present disclosure confers antibody-dependent cell cytotoxicity (ADCC) capacity to T lymphocytes and enhances ADCC in NK cells. When the receptor is engaged by an antibody (or another anti-tumor molecule comprising the Fc portion) bound to tumor cells, it triggers T-cell activation, sustained proliferation and specific cytotoxicity against cancer cells targeted by the antibody (or such other anti-tumor molecule comprising the Fc portion). As disclosed in the Examples section, below, T lymphocytes comprising the receptor of the present disclosure were highly cytotoxic against a wide range of tumor cell types, including B-cell lymphoma, breast and gastric cancer, neuroblastoma and osteosarcoma, as well as primary chronic lymphocytic leukemia (CLL). Cytotoxicity was entirely dependent on the presence of a specific antibody bound to target cells: soluble antibodies did not induce exocytosis of cytolytic granules and did not provoke non-specific cytotoxicity. The degree of affinity of CD16 for the Fc portion of Ig is a critical determinant of ADCC and thus to clinical responses to antibody immunotherapy. The CD16 with the 158V polymorphism was selected as an example; this variant has a high binding affinity for Ig and mediates superior ADCC.

The chimeric receptor of the present disclosure facilitates T-cell therapy by allowing one single receptor to be used for multiple cancer cell types. It also allows the targeting of multiple antigens simultaneously, a strategy that may ultimately be advantageous given immunoescape mechanism exploited by tumors (Grupp et al., N. Engl. J. Med. 2013; 368(16):1509-1518). Antibody-directed cytotoxicity could be stopped whenever required by simple withdrawal of antibody administration. Because the T cells expressing the chimeric receptor of the present disclosure are only activated by antibody bound to target cells, unbound immunoglobulin should not exert any stimulation on the infused T cells. Clinical safety can be further enhanced by using mRNA electroporation to express the chimeric receptor transiently, to limit any potential autoimmune reactivity.

The results disclosed in the Examples section, below, suggest that the infusion of autologous T cells, activated and expanded *ex vivo* and re-infused after genetic modification with the chimeric receptor of the present disclosure should significantly boost ADCC. Because the combined CD3ζ/4-1BB signaling also causes T-cell proliferation, there should be an accumulation of activated T cells at the tumor site which may further potentiate their activity.

Thus, in one option, the present disclosure provides a method for enhancing efficacy of an antibody-based immunotherapy of a cancer in a subject in need thereof, which subject is being treated with an antibody which can bind to cancer cells and has a humanized Fc portion which can bind to human CD16, said method comprising introducing into the subject a therapeutically effective amount of T lymphocytes or NK cells, which T lymphocytes or NK cells comprise the chimeric receptor of the present disclosure.

In another option, the present disclosure provides a method of enhancing T lymphocyte or NK cell ADCC activity in a subject comprising administering to the subject a T lymphocyte or NK cell, which T lymphocyte or NK cell comprises the chimeric receptor of the present disclosure. In one option, the subject has cancer. In one specific option, such subject is being treated with an antibody which can bind to cancer cells.

In one option of the above methods, the T lymphocytes or NK cells are autologous T lymphocytes or NK cells isolated from the subject. In one specific option, prior to re-introduction into the subject, the autologous T lymphocytes or NK cells are activated and/or expanded *ex vivo.* In another option, the T lymphocytes or NK cells are allogeneic T lymphocytes or NK cells. In one specific option, the T lymphocytes are allogeneic T lymphocytes in which the expression of the endogenous T cell receptor has been inhibited or eliminated. In one specific option, prior to introduction into the subject, the allogeneic T lymphocytes are activated and/or expanded *ex vivo.* T lymphocytes can be activated by any method known in the art, e.g., in the presence of anti-CD3/CD28, IL-2, and/or phytohemoagglutinin. NK cells can be activated by any method known in the art, e.g., in the presence of one or more agents selected from the group consisting of CD137 ligand protein, CD137 antibody, IL-15 protein, IL-15 receptor antibody, IL-2 protein, IL-12 protein, IL-21 protein, and K562 cell line. See, e.g., U.S. Patents Nos. 7,435,596 and 8,026,097 for the description of useful methods for expanding NK cells.

In one option of the above methods, the chimeric receptor is introduced into the T lymphocytes or the NK cells (*e.g.,* after *ex vivo* activation and/or expansion) by retroviral transduction, lentiviral transduction, or DNA or RNA electroporation.

In one option of the above methods, introduction (or re-introduction) of T lymphocytes or NK cells to the subject is followed by administering to the subject a therapeutically effective amount of IL-2.

The chimeric receptor of the present disclosure may be used for treatment of any cancer, including, without limitation, carcinomas, lymphomas, sarcomas, blastomas, and leukemias, for which a specific antibody with an Fc portion that binds to CD16 exists or is capable of being generated. Specific non-limiting examples of cancers, which can be treated by the chimeric receptor of the present disclosure include, e.g., cancers of B-cell origin (e.g., B-lineage acute lymphoblastic leukemia, B-cell chronic lymphocytic leukemia and B-cell non-Hodgkin's lymphoma), breast cancer, gastric cancer, neuroblastoma, and osteosarcoma.

Non-limiting examples of anti-cancer antibodies containing an Fc portion that can bind to human CD16, whose efficacy can be enhanced by the method of the present disclosure, include, for example, Rituximab, Trastuzumab, hu14.18K322A, Epratuzumab, Cetuximab, and Labetuzumab.

The appropriate dosage of the antibody used will depend on the type of cancer to be treated, the severity and course of the disease, previous therapy, the patient's clinical history and response to the antibody, and the discretion of the attending physician. The antibody can be administered to the patient at one time or over a series of treatments. The progress of the therapy of the present disclosure can be easily monitored by conventional techniques and assays.

The administration of antibodies can be performed by any suitable route, including systemic administration as well as administration directly to the site of the disease (*e.g.,* to primary tumor).

The T lymphocytes used in the methods of the present disclosure are most preferably patient's own cells (i.e., autologous cells) that were earlier isolated from a blood sample and preferably activated and expanded *ex vivo* (e.g., for 3-5 days) with standard methods, such as, e.g., anti-CD3/CD28 beads, IL-2, or phytohemoagglutinin. Alternatively, allogeneic T lymphocytes can be used (preferably allogeneic T lymphocytes in which the expression of the endogenous T cell receptor has been inhibited or eliminated). See Torikai et al., Blood, 2012 119: 5697-5705. Following isolation (and optionally activation and/or expansion), T lymphocytes and NK cells from a patient are transduced (or electroporated) with the polynucleotide encoding the chimeric receptor of the present disclosure (or a vector comprising such polynucleotide) so that the chimeric receptor is expressed on the cell surface of the T cell or NK cell. The modified cells can then be administered into the patient (e.g., 1 day after infusion of a therapeutic antibody).

In accordance with the present disclosure, patients can be treated by infusing therapeutically effective doses of T lymphocytes or NK cells comprising the chimeric receptor of the present disclosure in the range of about 10⁵ to 10¹⁰ or more cells per kilogram of body weight (cells/Kg). The infusion can be repeated as often and as many times as the patient can tolerate until the desired response is achieved. The appropriate infusion dose and schedule will vary from patient to patient, but can be determined by the treating physician for a particular patient. Typically, initial doses of approximately 10⁶ cells/Kg will be infused, escalating to 10⁸ or more cells/Kg. IL-2 can be co-administered to expand infused cells post-infusion. The amount of IL-2 can about 1-5 x 10⁶ international units per square meter of body surface.

NK cells used in the methods of the present disclosure may be preferentially expanded by exposure to cells that lack or poorly express major histocompatibility complex I and/or II molecules and which have been genetically modified to express membrane bound IL-15 and 4-1BB ligand (CDI37L). Such cell lines include, but are not necessarily limited to, K562 [ATCC, CCL 243; Lozzio et al., Blood 45(3): 321-334 (1975); Klein et al., Int. J. Cancer 18: 421-431 (1976)], and the Wilms tumor cell line HFWT. [Fehniger T A, Caligiuri M A. Int Rev Immunol 20(3-4):503-534 (2001); Harada H, et al., Exp Hematol 32(7):614-621 (2004)], the uterine endometrium tumor cell line HHUA, the melanoma cell line HMV-II, the hepatoblastoma cell line HuH-6, the lung small cell carcinoma cell lines Lu-130 and Lu-134-A, the neuroblastoma cell lines NB 19 and N1369, the embryonal carcinoma cell line from testis NEC 14, the cervix carcinoma cell line TCO-2, and the bone marrow-metastasized neuroblastoma cell line TNB 1 [Harada H., et al., Jpn. J. Cancer Res 93: 313-319 (2002)]. Preferably the cell line used lacks or poorly expresses both MHC I and II molecules, such as the K562 and HFWT cell lines. A solid support may be used instead of a cell line. Such support should preferably have attached on its surface at least one molecule capable of binding to NK cells and inducing a primary activation event and/or a proliferative response or capable of binding a molecule having such an affect thereby acting as a scaffold. The support may have attached to its surface the CD137 ligand protein, a CD137 antibody, the IL-15 protein or an IL-15 receptor antibody. Preferably, the support will have IL-15 receptor antibody and CD137 antibody bound on its surface.

### Combination Treatments of the Present disclosure

The compositions and methods described in the present disclosure may be utilized in conjunction with other types of therapy for cancer, such as chemotherapy, surgery, radiation, gene therapy, and so forth. Such therapies can be administered simultaneously or sequentially (in any order) with the immunotherapy according to the present disclosure.

When co-administered with an additional therapeutic agent, suitable therapeutically effective dosages for each agent may be lowered due to the additive action or synergy.

The treatments of the present disclosure can be combined with other immunomodulatory treatments such as, e.g., therapeutic vaccines (including but not limited to GVAX, DC-based vaccines, etc.), checkpoint inhibitors (including but not limited to agents that block CTLA4, PD1, LAG3, TIM3, etc.) or activators (including but not limited to agents that enhance 41BB, OX40, etc.).

Non-limiting examples of other therapeutic agents useful for combination with the immunotherapy of the present disclosure include:
(i) anti-angiogenic agents (e.g., TNP-470, platelet factor 4, thrombospondin-1, tissue inhibitors of metalloproteases (TIMP1 and TIMP2), prolactin (16-Kd fragment), angiostatin (38-Kd fragment of plasminogen), endostatin, bFGF soluble receptor, transforming growth factor beta, interferon alpha, soluble KDR and FLT-1 receptors, placental proliferin-related protein, as well as those listed by Carmeliet and Jain (2000));
(ii) a VEGF antagonist or a VEGF receptor antagonist such as anti-VEGF antibodies, VEGF variants, soluble VEGF receptor fragments, aptamers capable of blocking VEGF or VEGFR, neutralizing anti-VEGFR antibodies, inhibitors of VEGFR tyrosine kinases and any combinations thereof;
(iii) chemotherapeutic compounds such as, e.g., pyrimidine analogs (5-fluorouracil, floxuridine, capecitabine, gemcitabine and cytarabine), purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethyhnelamineoxaliplatin, iphosphamide, melphalan, merchlorehtamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes-dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (*e.g.,* TNP-470, genistein, bevacizumab) and growth factor inhibitors (e.g., fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; and chromatin disruptors.

For examples of additional useful agents see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

Without further elaboration, it is believed that one skilled in the art can, based on the above description, utilize the present invention to its fullest extent. The following specific options are, therefore, to be construed as merely illustrative, and not limitative of the remainder of the disclosure in any way whatsoever.

### EXAMPLES

The present disclosure is also described and demonstrated by way of the following examples. However, the use of these and other examples anywhere in the specification is illustrative only and in no way limits the scope and meaning of the present disclosure or of any exemplified term. Likewise, the present disclosure is not limited to any particular preferred options described here. Indeed, many modifications and variations of the present disclosure may be apparent to those skilled in the art upon reading this specification.

### MATERIALS AND METHODS

### Cells

The human B-lineage lymphoma cell lines Daudi and Ramos, the T-cell acute lymphoblastic leukemia cell line Jurkat, and the neuroblastoma cell lines CHLA-255, NB1691 and SK-N-SH were available at St. Jude Children's Research Hospital. The breast carcinoma cell lines MCF-7 (ATCC HTB-22) and SK-BR-3 (ATCC HTB-30), and the osteosarcoma cell line U-2 OS (ATCC HTB-96) were obtained from the American Type Culture Collection (ATCC; Rockville, MD); the gastric carcinoma cell line MKN7 was from National Institute of Biomedical Innovation (Osaka, Japan). Daudi, CHLA-255, NB1691, SK-N-SH, SK-BR-3, MCF-7, U-2 OS and MKN7 were also transduced with a murine stem cell virus (MSCV)-internal ribosome entry site (IRES)-green fluorescent protein (GFP) retroviral vector containing the firefly luciferase gene (Fujisaki et al., Cancer Res. 2009; 69(9):4010-4017). Transduced cells were selected for their expression of GFP with a FACSAria cell sorter (BD Biosciences, San Jose, CA). Peripheral blood or bone marrow samples from newly diagnosed and untreated patients with B-chronic lymphocytic leukemia (CLL) were obtained following informed consent and approval from the Domain Specific Ethics Board governing Singapore's National University Hospital.

Peripheral blood samples were obtained from de-identified by-products of platelet donations from healthy adult donors. Mononuclear cells were enriched by centrifugation on Accu-Prep Human Lymphocytes Cell Separation Media (Accurate Chemical & Scientific Corp., Westbury, N.Y.), and cultured with anti-CD3/CD28 beads (Invitrogen, Carlsbad, CA) in RPMI-1640 with 10% fetal bovine serum (FBS), antibiotics, 100 IU interleukin (IL)-2 (Roche, Mannheim, Germany) for 3days. On day 4, T cells were purified by negative selection with a mixture of CD14, CD16, CD19, CD36, CD56, CD123 and CD235a antibodies and magnetic beads (Pan T Cell Isolation Kit II; Miltenyi Biotec, Bergisch Gladbach, Germany) (purity, >98%). Purified T cells were maintained in the above medium, with the addition of 100 IU IL-2 every other day.

### Plasmids, virus production and gene transduction

The pMSCV-IRES-GFP, pEQ-PAM3(-E), and pRDF were obtained from the St. Jude Children's Research Hospital Vector Development and Production Shared Resource (Memphis, TN).¹⁰ The *FCRG3A* cDNA was obtained from Origene (Rockville, MD) and its V158F variant was generated using site-directed mutagenesis by PCR using primers "F" CTTCTGCAGGGGGCTTGTTGGGAGTAAAAATGTGTC (SEQ ID NO: 7) and "R" GACACATTTTTACTCCCAACAAGCCCCCTGCAGAAG (SEQ ID NO: 8). The polynucleotides encoding CD8α hinge and transmembrane domain (SEQ ID NO: 11), and the intracellular domains of 4-1BB (SEQ ID NO: 12) and CD3ζ (SEQ ID NO: 13) were subcloned from an anti-CD19-41BB-CD3ζ cDNA previously made. See Imai et al., Leukemia 2004; 18:676-684. These molecules were assembled using splicing by overlapping extension by PCR. The constructs ("CD16F-BB-ζ" and "CD16V-BB-ζ") and the expression cassette were subcloned into EcoRI and MLu1 sites of the MSCV-IRES-GFP vector.

To generate RD114-pseudotyped retrovirus, fuGENE 6 or X-tremeGENE 9 (Roche, Indianapolis, IN) was used to transfect 3 x 10⁶ 293T cells with 3.5 µg of cDNA encoding CD16V-BB-ζ, 3.5 µg of pEQ-PAM3(-E), and 3 µg of pRDF (Imai et al., Leukemia 2004; 18:676-684). After replacing the medium with RPMI-1640 with 10% FBS at 24 hours, the medium containing retrovirus was harvested after 48-96 hours and added to RetroNectin (TakaRa, Otsu, Japan)-coated polypropylene tubes, which were centrifugated at 1400 g for 10 min and incubated at 37°C for 6 hours. After additional centrifugation, and removal of the supernatant, T cells (1 x 10⁵) were added to the tubes and left in at 37°C for 24 hours. Cells were then maintained in RPMI-1640 with FBS, antibiotics and 100 IU/mL IL-2 until the time of the experiments, 7-21 days after transduction.

Surface expression of CD16 was analyzed by flow cytometry using R-Phycoerythrin conjugated anti-human CD16 (clone B73.1, BD Biosciences Pharmingen, San Diego, CA). For western blotting, 2 x 10⁷ T cells were lysed in Cellytic M lysis Buffer (Sigma, St Louis, MO) containing 1% protease inhibitor cocktail (Sigma) and 1% phosphatase inhibitor cocktail 2 (Sigma). After centrifugation, lysate supernatants were boiled with an equal volume of LDS buffer (Invitrogen, Carlsbad, CA) with or without reducing buffer (Invitrogen) and then separated by NuPAGE Novex 12% Bis-Tris Gel (Invitrogen). The proteins were transferred to a polyvinylidene fluoride (PVDF) membrane, which was incubated with a mouse anti-human CD3ζ (clone 8D3; BD eBioscience Pharmingen) and then with a goat anti-mouse IgG horseradish peroxidase-conjugated secondary antibody (Cell Signaling Technology, Danvers, MA). Antibody binding was revealed by using the Amersham ECL Prime detection reagent (GE Healthcare).

### mRNA electroporation

The pVAX1 vector (Invitrogen, Carlsbad, CA) was used as a template for in vitro mRNA transcription. The CD16V-BB-ζ cDNA was subcloned into EcoRI and XbaI sites of pVAX1. The corresponding mRNA was transcribed in vitro with T7 mScript mRNA production system (CellScript, Madison, WI). See, *e.g.,* Shimasaki et al., Cytotherapy. 2012;14(7):830-40.

For electroporation, the Amaxa Nucleofector (Lonza, Walkersville, MD) was used; 1 x 10⁷ of purified T cells activated with 200 IU/mL IL-2 overnight were mixed with 200 µg/mL mRNA in Cell Line Nucleofector Kit V (Lonza), transferred into the processing chamber, and transfected using the program X-001. Immediately after electroporation, cells were transferred from the processing chamber into a 24-well plate and then cultured in RPMI-1640 with FBS, antibiotics and 100 IU/mL IL-2 (Roche, Mannheim, Germany). See also Shimasaki et al., Cytotherapy, 2012, 1-11.

### Antibody binding, cell conjugation and cell proliferation assays

To measure the chimeric receptors' antibody-binding capacity, T lymphocytes (5 x 10⁵) transduced with chimeric receptors or a vector containing GFP only were incubated with Rituximab (Rituxan, Roche; 0.1-1 µg/mL), Trastuzumab (Herceptin; Roche; 0.1-1 µg/mL) and/or purified human IgG (R&D Systems, Minneapolis, MN ; 0.1-1 µg/mL) for 30 minutes at 4°C. After washing twice with phosphate buffered saline (PBS), cells were incubated with goat anti-human IgG-PE (Southern Biotechnology Associates, Birmingham, AL) for 10 minutes at room temperature and cell staining was measured using an Accuri C6 flow cytometer (BD Biosciences).

To determine whether antibody binding to the receptor promoted cell aggregation, CD20-positive Daudi cells were labeled with CellTrace calcein red-orange AM (Invitrogen) and then incubated with Rituximab (0.1 µg/mL) for 30 minutes at 4°C. After washing twice in PBS, cells with Jurkat cells transduced with the chimeric receptor or mock-transduced at 1:1 E:T ratio in 96 round bottom plates (Costar, Corning, NY) for 60 min at 37°C. The proportion of cells forming heterologous cell aggregates (calcein AM-GFP double positive) was determined by flow cytometry.

To measure cell proliferation, 1 x 10⁶ of T cells transduced with the chimeric receptor or mock-transduced were placed in the wells of a 24-well plate (Costar, Corning, NY) in RPMI-1640 with FBS, antibiotics and 50 IU/mL IL-2. Daudi cells were treated with Streck cell preservative (Streck Laboratories, Omaha, NE) to stop proliferation and labeled with Rituximab (0.1 µg/mL) for 30 min at 4°C. They were added to the wells, at 1:1 ratio with T cells, on days 0, 7, 14 and 21. The n number of viable T cells after culture was measured by flow cytometry.

### CD107 degranulation and cytotoxicity assays

To determine whether CD16 cross-linking caused exocytosis of lytic granules, chimeric receptor- and mock transduced T cells (1 x 10⁵) were placed into each well of a Rituximab-coated 96-well flat bottom plate and cultured for 4 hours at 37°C. In other experiments, T cells were co-cultured with Daudi cells pre-incubated with Rituximab. An anti-human CD107a antibody conjugated to phycoerythrin (BD Biosciences) was added at the beginning of the cultures and one hour later GolgiStop (0.15 µl; BD Biosciences) was added. CD107a positive T cells were analyzed by flow cytometry.

To test cytotoxicity, target cells were suspended in RPMI-1640 with 10% FBS, labeled with calcein AM (Invitrogen) and plated into 96-well round bottom plates (Costar). T cells were added at various E: T ratio as indicated in Results, and co-cultured with target cells for 4 hours, with or without the antibodies Rituximab (Rituxan, Roche), Trastuzumab (Herceptin, Roche), or hu14.18K322A (obtained from Dr. James Allay, St Jude Children's GMP, Memphis, TN; at µg/mL). At the end of the cultures, cells were collected, resuspended in an identical volume of PBS, propidium iodide was added. The number of viable target cells (calcein AM-positive, propidium-iodide negative) was counted using the Accuri C6 flow cytometer (Fujisaki et al., Cancer Res. 2009; 69(9):4010-4017). For adherent cell lines, cytotoxicity was tested using luciferase-labeled target cells. To measure cytotoxicity against the adherent cell lines NB1691, CHLA-255, SK-BR-3, MCF-7, U-2 OS and MKN7, their luciferase-labeled derivatives were used. After plating for at least 4 hours, T cells were added as described above. After 4 hours of co-culture, the Promega Bright-Glo luciferase reagent (Promega, Madison, WI) was added to each well; 5 minutes later, luminescence was measured using a plate reader Biotek FLx800 (BioTek, Tucson, AZ) and analyzed with the Gen5 2.0 Data Analysis Software.

### Xenograft experiments

Daudi cells expressing luciferase were injected intraperitoneally (i.p.; 0.3 x 10⁶ cells per mouse) in NOD.Cg-Prkdc^{scid} IL2rg^{tm1Wjl}/SzJ (NOD/scid IL2RGnull) mice (Jackson Laboratory, Bar Harbor). Some mice received Rituximab (100 µg) i.p. 4 days after Daudi inoculation, with or without i.p. injection of human primary T cells on days 5 and 6. T cells had been activated with anti-CD3/CD28 beads for 3 days, transduced with the CD16V-BB-ζ receptor, resuspended in RPMI-1640 plus 10% FBS and then injected at 1x 10⁷ cells per mouse. Rituximab injection was repeated weekly for 4 weeks, with no further T lymphocyte injection. All mice received i.p. injections of 1000-2000 IU of IL-2 twice a week for 4 weeks. A group of mice received tissue culture medium instead of Rituximab or T cells.

Tumor engraftment and growth was measured using a Xenogen IVIS-200 system (Caliper Life Sciences, Hopkinton, MA). Imaging commenced 5 minutes after i.p. injection of an aqueous solution of D-luciferin potassium salt (3 mg/mouse) and photons emitted from luciferase-expressing cells were quantified using the Living Image 3.0 software.

### RESULTS

### Expression of the CD16V-BB-ζ receptor

The V158 polymorphism of *FCRG3A* (*CD16*), expressed in about one-fourth of individuals, encodes a high-affinity immunoglobulin Fc receptor and is associated with favorable responses to antibody therapy (25, 26, 29-31). A V158 variant of the *FCGR3A* gene was generated. It was combined with the hinge and transmembrane domain of CD8α, the T-cell stimulatory molecule CD3ζ, and the co-stimulatory molecule 4-1BB (Fig. 1A). Imai et al., Leukemia 2004; 18:676-684 . An MCSV retroviral vector containing the CD16V-BB-ζ construct and GFP was used to transduce peripheral blood T lymphocytes from 12 donors: median GFP expression in CD3+ cells was 89.9% (range, 75.3%-97.1%); in the same cells, median chimeric receptor surface expression as assessed by anti-CD16 staining was 83.0% (67.5%-91.8%) (Fig. 1B). T lymphocytes from the same donors transduced with a vector containing only GFP had a median GFP expression of 90.3% (67.8%-98.7%) but only 1.0% (0.1%-2.7%) expressed CD16 (Fig. 1B). Expression of the receptor did not differ significantly between CD4+ and CD8+ T cells: 69.8% ± 10.8% CD4+ cells were CD16+ after transduction with CD16V-BB-ζ, as compared to 77.6% ± 9.2% CD8+ cells (Fig. 2).

To ensure that the other components of the chimeric receptor were expressed, levels of expression of CD3ζ were measured by flow cytometry. As shown in Fig. 1B, CD16V-BB-ζ-transduced T lymphocytes expressed CD3ζ at levels much higher than those expressed by mock-transduced cells: mean (± SD) of the mean fluorescence intensity was 45,985 ± 16,365 in the former versus 12,547 ± 4,296 in the latter (P = 0.027 by *t* test; n = 3; Fig. 1B). The presence of the chimeric protein was also determined by western blotting probed with the anti-CD3ζ antibody. As shown in Fig. 1C, CD16V-BB-ζ -transduced T lymphocytes expressed a chimeric protein of approximately 25 kDa under reducing conditions, in addition to the endogenous CD3ζ of 16 kDa. Under non-reducing conditions, the CD16V-BB-ζ protein was shown to be expressed as either a monomer or a dimer of 50 kDa.

### Antibody-binding capacity of V158 versus F158 CD16 receptors

To test the capacity of the CD16V-BB-ζ chimeric receptor to bind immunoglobulin (Ig), peripheral blood T lymphocytes from 3 donors were transduced. As shown in Fig. 3A, CD16V-BB-ζ-expressing T lymphocytes were coated with the antibody after incubation with Rituximab. Similar results were obtained with Trastuzumab and human IgG.

The Ig-binding capacity of the CD16V-BB-ζ receptor, which contained the high-affinity V158 polymorphism of *FCRG3A* (*CD16*), was then compared to that of an identical receptor containing the F158 variant instead ("CD16F-BB-ζ"). After transducing Jurkat cells with either receptor, they were incubated with Rituximab and an anti-human Ig PE antibody (binding Rituximab) and the PE fluorescence intensity was related to that of GFP. As shown in Fig. 3B, at any given level of GFP, cells transduced with the CD16V-BB-ζ receptor had a higher PE fluorescence intensity than that of cells transduced with the CD16F-BB-ζ receptor, indicating that the former had a significantly higher antibody-binding affinity. Trastuzumab and human IgG were also bound by CD16V-BB-ζ receptors with a higher affinity (Fig. 4).

To determine whether antibody binding to the CD16V-BB-ζ receptor could promote aggregation of effector and target cells, Jurkat cells expressing CD16V-BB-ζ (and GFP) were mixed at a 1:1 ratio with the CD20⁺ Daudi cell line (labeled with Calcein AM red-orange) for 60 minutes, and the formation of GFP-Calcein doublets was measured with or without addition of Rituximab. In 3 experiments, 39.0% ± 1.9% of events in the coculture were doublets if Jurkat cells expressed CD16V-BB-ζ receptors and Rituximab was present (Fig. 3C and D). By contrast, there were <5% doublets with human IgG instead of Rituximab, or with mock-transduced Jurkat cells regardless of whether Rituximab was present.

### Binding of Ig to CD16V-BB-ζ induces T cell activation, degranulation and cell proliferation

It was assessed whether CD16V-BB-ζ receptor cross-linking by an immobilized antibody could induce activation signals in T lymphocytes. Indeed, T lymphocytes transduced with CD16V-BB-ζ markedly increased IL-2 receptor expression (CD25) when cultured on plates coated with Rituximab whereas no changes were detected in the absence of antibody, or in mock-transduced cells regardless of whether the antibody was present (Fig. 5A and B).

In addition to expression of IL-2 receptors, CD16V-BB-ζ receptor cross-linking triggered exocytosis of lytic granules in T lymphocytes, as detected by CD107a staining. Thus, in 6 experiments in which T lymphocytes from 4 donors were either seeded onto microtiter plates coated with Rituximab (n = 3) or cocultured with Daudi cells in the presence of Rituximab (n = 3), T lymphocytes expressing CD16V-BB-ζ became CD107a positive (Fig. 5C).

Finally, it was determined whether receptor cross-linking could induce cell proliferation. As shown in Fig. 5D, T lymphocytes expressing CD16V-BB-ζ expanded in the presence of Rituximab and Daudi cells (at a 1 : 1 ratio with T lymphocytes): in 3 experiments, mean T cell recovery after 7 days of culture was 632% (± 97%) of input cells; after 4 weeks of culture, it was 6877% (± 1399%). Of note, unbound Rituximab, even at a very high concentration (1-10 µg/mL), had no significant effect on cell proliferation in the absence of target cells, and no cell growth occurred without Rituximab, or in mock-transduced T cells regardless of the presence of the antibody and/or target cells (Fig. 5D). Thus, CD16V-BB-ζ receptor cross-linking induces signals that result in sustained proliferation.

### T lymphocytes expressing CD16V-BB-ζ mediate ADCC in vitro and in vivo

The observation that CD16V-BB-ζ cross-linking provoked exocytosis of lytic granules implied that CD16V-BB-ζ T lymphocytes should be capable of killing target cells in the presence of specific antibodies. Indeed, in 4-hour in vitro cytotoxicity assays, CD16V-BB-ζ T lymphocytes were highly cytotoxic against the B-cell lymphoma cell lines Daudi and Ramos in the presence of Rituximab: more than 50% target cells were typically lysed after 4 hours of co-culture at a 2 : 1 E : T ratio (Fig. 6 and Fig. 7). By contrast, target cell killing was low in the absence of the antibody or with mock-transduced T cells (Fig. 6 and Fig. 7). Notably, the effector cells used in these experiments were highly enriched with CD3+ T lymphocytes (>98%) and contained no detectable CD3-- CD56+ NK cells. Rituximab-mediated cytotoxicity of CD16V-BB-ζ T lymphocytes was also clear with CD20+ primary CLL cells (n = 5); as shown in Fig. 6B, cytotoxicity typically exceeded 70% after 4 hours of coculture at a 2:1 E:T ratio. Bone marrow mesenchymal stromal cells have been shown to exert immunosuppressive effects (Jefferis, Nat. Rev. Drug Discov. 2009; 8(3):226-234; Clemenceau et al., Blood 2006; 107(12):4669-4677). To test whether this would affect the cytotoxic capacity of CD16V-BB-ζ T lymphocytes, they were co-cultured with CLL cells in the presence of bone marrow-derived mesenchymal stromal cells for 24 hours at a 1:2 E:T. As shown in Fig. 6C, mesenchymal cells did not diminish the killing capacity of the ADCC-mediating lymphocytes.

Next, it was determined whether different immunotherapeutic antibodies could trigger similar cytotoxicity against tumor cells expressing the corresponding antigen. Thus, the cytotoxicity of CD16V-BB-ζ T lymphocytes was tested against solid tumor cells expressing HER2 (the breast cancer cell lines MCF-7 and SK-BR-3 and the gastric cancer cell line MKN7) or GD2 (the neuroblastoma cell lines CHLA-255, NB1691 and SK-N-SH, and the osteosarcoma cell line U2-OS). The antibodies Trastuzumab were used to target HER2 and hu14.18K322A were used to target GD2. CD16V-BB-ζ T lymphocytes were highly cytotoxic against these cells in the presence of the corresponding antibody (Fig. 6 and Fig. 7). In experiments with NB1691, it was also tested whether cytotoxicity could be achieved at even lower E:T ratios by prolonging the culture to 24 hours. As shown in Fig.8, cytotoxicity exceeded 50% at 1:8 ratio in the presence of hu14.18K322A. To further test the specificity of the CD16V-BB-ζ-mediated cell killing, the CD20+ Daudi cells were cultured with CD16V-BB-ζ T lymphocytes and antibodies of different specificity: only Rituximab mediated cytotoxicity, while there was no increase in cytotoxicity in the presence of Trastuzumab or hu14.18K322A (Fig. 8). Finally, it was determined whether CD 16V-BB-ζ-mediated cell killing in the presence of immunotherapeutic antibodies could be inhibited by unbound monomeric IgG. As shown in Fig. 8, T cell cytotoxicity was not affected even if IgG was present at up to 1000 times higher concentration than the cell-bound immunotherapeutic antibody.

To gauge the anti-tumor capacity of CD16V-BB-ζ T lymphocytes in vivo, experiments with NOD/scid IL2RGnull mice engrafted with luciferase-labeled Daudi cells were performed. Tumor growth was measured by live imaging in mice receiving CD16V-BB-ζ T lymphocytes plus Rituximab, and their outcome was compared to mice receiving either Rituximab or T lymphocytes alone, or no treatment. As shown in Fig. 9, tumor cells expanded in all mice except those that received Rituximab followed by CD16V-BB-ζ T lymphocytes. All 5 mice treated with this combination were still in remission >120 days after tumor injection, in contrast to 0 of 12 mice that were untreated or received antibody or cells alone. A strong anti-tumor activity was also observed in mice engrafted with the neuroblastoma cell line NB1691 and treated with hu14.18K322A and CD16V-BB-ζ T lymphocytes (Fig. 10).

### Comparison of CD16V-BB-ζ with other receptors

The function of T cells bearing either CD16V-BB-ζ or CD16F-BB-ζ receptors was compared. In line with their higher affinity for Ig, CD16V-BB-ζ receptors induced significantly higher T cell proliferation and ADCC than that triggered by the lower affinity CD16F-BB-ζ receptors (Fig. 11).

Next, the function of T cells bearing CD16V-BB-ζ was compared to that of T cells expressing other receptors with different signaling properties. These included a receptor with no signaling capacity ("CD16V-truncated"), one with CD3ζ but no 4-1BB ("CD16V-ζ"), and a previously described receptor that combined CD16V with the transmembrane and cytoplasmic domains of FcεRIγ ("CD16V-FcεRIγ") (Jefferis, Nat. Rev. Drug Discov. 2009; 8(3):226-234; Clemenceau et al., Blood 2006; 107(12):4669-4677) (Fig. 12). After retroviral transduction in activated T cells, all receptors were highly expressed (Fig. 13). As shown in Fig. 14, CD16V-BB-ζ induced significantly higher activation, proliferation and specific cytotoxicity than all other constructs.

### Expression of CD16V-BB-ζ receptors by mRNA electroporation

In all the above experiments, CD 16V-BB-ζ expression was enforced by retroviral transduction. It was tested whether an alternative method, electroporation of mRNA, could also confer ADCC capacity to T lymphocytes. Activated T lymphocytes from 2 donors were electroporated and high expression efficiencies were obtained: 55% and 82% of T lymphocytes became CD16+ 24 hours after electroporation (Fig. 15A). In the second donor, receptor expression was also tested on day 3, when it was 43%, a result similar to those of previous experiments with another receptor where expression persisted for 72 to 96 hours (Fujisaki et al., Cancer Res. 2009; 69(9):4010-4017). ADCC was activated in T lymphocytes electroporated with CD16V-BB-ζ mRNA: in the presence of Rituximab, 80% Ramos cells were killed after 4 hours at a 2 : 1 E : T ratio, while cells electroporated without mRNAs were ineffective (Fig. 15B).

### DISCUSSIONS

Described herein is the development of a chimeric receptor which endows T lymphocytes with the capacity to exert ADCC. When the CD16V-BB-ζ receptor is engaged by an antibody bound to tumor cells, it triggers T-cell activation, sustained proliferation and specific cytotoxicity against cancer cells targeted by antibody. CD16V-BB-ζ T lymphocytes were highly cytotoxic against a wide range of tumor cell types, including B-cell lymphoma, breast and gastric cancer, neuroblastoma and osteosarcoma, as well as primary CLL cells. Cytotoxicity was entirely dependent on the presence of a specific antibody bound to target cells; unbound antibodies did not provoke non-specific cytotoxicity nor affected cytotoxicity with cell-bound antibodies. CD16V-BB-ζ T cells also killed CLL cells when these were cultured on mesenchymal cell layers, regardless of the known immunosuppressive effects of this microenvironment (Jefferis, Nat. Rev. Drug Discov. 2009; 8(3):226-234; Clemenceau et al., Blood 2006; 107(12):4669-4677). Moreover, CD16V-BB-ζ T lymphocytes infused after Rituximab eradicated B-cell lymphoma cells engrafted in immunodeficient mice, and had considerable anti-tumor activity in mice engrafted with neuroblastoma cells in the presence of an anti-GD2 antibody. In sum, T cells expressing CD16V-BB-ζ effected strong ADCC in vitro and in vivo.

The affinity of CD16 for the Fc portion of Ig is a critical determinant of ADCC and, thus, influences clinical responses to antibody immunotherapy. Hence, considerable efforts are being made to further enhance the affinity of Fc fragments for FcγR, for example by glycoengineering (Nimmerjahn et al., Nat. Rev. Immunol. 2008; 8(1):34-47, Kohrt et al., Immunotherapy 2012; 4(5):511-527). To construct the chimeric receptor of the present disclosure, the *FCRG3A* (*CD16*) gene with the 158V polymorphism (SEQ ID NO: 10) was used as an example. This variant encodes a receptor with higher binding affinity for Ig and has been shown to mediate superior ADCC (Ferris et al., J. Clin. Oncol. 2010; 28(28):4390-4399; Koene et al., Blood 1997; 90(3):1109-1114; Cartron et al., Blood 2002; 99(3):754-758; Weng et al., J. Clin. Oncol. 2003; 21(21):3940-3947; Dall'Ozzo et al., Cancer Res. 2004; 64(13):4664-4669; Hatjiharissi et al., Blood 2007; 110(7):2561-2564; Musolino et al., J. Clin. Oncol. 2008; 26(11): 1789-1796; Bibeau et al., J. Clin. Oncol. 2009; 27(7): 1122-1129; Ahlgrimm et al., Blood 2011; 118(17):4657-4662; Veeramani et al., Blood 2011; 118(12):3347-3349). Indeed, in side-to-side comparisons with a chimeric receptor containing the more common F158 variant, the CD16V-BB-ζ had a significantly higher capacity to bind human Ig Fc, and induced more vigorous proliferation and cytotoxicity, evoking results of recent studies addressing the role of affinity in chimeric antigen receptor function (Kono et al., Cancer Res. 2002; 62(20):5813-5817; Delgado et al., Cancer Res. 2010; 70(23):9554-956). Current "second generation" chimeric receptors combine a stimulatory molecule with a co-stimulatory one to augment signaling and prevent activation-induced apoptosis. Therefore, CD16 V158 was combined with a stimulatory molecular tandem constituted by CD3ζ and 4-1BB (CD137). Indeed, the CD16V-BB-ζ receptor induced a markedly superior T cell activation, proliferation and cytotoxicity than did receptors acting through CD3ζ alone, or of FcεRIγ.

The clinical potential of genetically modified T cells expressing receptors that recognize antigens of the surface of tumor cells and can transduce stimulatory signals is being increasingly demonstrated by results of clinical trials (Pule et al., Nat. Med. 2008; 14(11):1264-1270; Porter et al., OncLive 2011; 25;365(8):725-733; Brenjens et al., Blood 2011; 118(18):4817-4828; Till et al., Blood 2012; 119(17):3940-3950; Kochenderfer et al., Blood 2012; 119(12):2709-2720; Brentjens et al., Sci. Transl. Med. 2013; 5(177):177ra138). Most notably, significant tumor reductions and/or complete remissions have been reported in patients with B-cell malignancies who received autologous T lymphocytes expressing chimeric antigen receptors against CD 19 or CD20 by viral transduction (Porter et al., OncLive 2011; 25;365(8):725-733; Brenjens et al., Blood 2011; 118(18):4817-4828; Till et al., Blood 2012; 119(17):3940-3950; Kochenderfer et al., Blood 2012; 119(12):2709-2720; Brentjens et al., Sci. Transl. Med. 2013; 5(177):177ra138). Expanding this strategy to other tumors involves considerable effort, including the development of another chimeric antigen receptor construct, and the optimization of large-scale transduction conditions in compliance with regulatory requirements. In this regard, the CD16V-BB-ζ receptor described hereinshould facilitate the implementation of T-cell therapy by allowing one single receptor to be used for multiple cancer cell types. It should also allow the targeting of multiple antigens simultaneously, a strategy that may ultimately be advantageous given immunoescape mechanisms exploited by tumors, as illustrated by the recent report of a leukemia relapse driven by a subclone lacking the marker targeted by a chimeric receptor with single specificity. Antibody-directed cytotoxicity could be stopped whenever required by simple withdrawal of antibody administration. Because the T cells expressing CD16V-BB-ζ are only activated by antibody bound to target cells, soluble immunoglobulin should not exert any stimulation on the infused T cells. Nevertheless, it would be important to test the clinical safety of this strategy by using mRNA electroporation to express the CD16V-BB-ζ receptor transiently, thus limiting any potential autoimmune reactivity. As demonstrated herein, mRNA electroporation can express the receptor very effectively.

Antibody therapy has become standard-of-care for many cancer subtypes; its clinical efficacy is mostly determined by its capacity to trigger ADCC through the engagement of Fc receptors (Ferris et al., J. Clin. Oncol. 2010; 28(28):4390-4399). The main effectors of ADCC are NK cells but their function can be impaired in patients with cancer. For example, it was reported that Trastuzumab-mediated ADCC of gastric cancer cells overexpressing HER2 was significantly lower with peripheral blood mononuclear cells from gastric cancer patients and advanced disease as compared to that obtained with samples from patients with early disease or healthy donors. Moreover, responses are likely to be influenced by other factors, including the genotype of NK-cell inhibitory receptors and their ligands. The results presented herein suggest that the infusion of autologous T cells genetically engineered with the CD16V-BB-ζ receptor should significantly boost ADCC. Because the combined CD3ζ/4-1BB signaling also causes T-cell proliferation, there should be an accumulation of activated T cells at the tumor site which may further potentiate their activity. CD16V-BB-ζ receptors can be expressed by mRNA electroporation not only in activated T lymphocytes but also in resting peripheral blood mononuclear cells, a procedure that would take only a few hours from blood collection to infusion of CD16V-BB-ζ-expressing cells and is therefore well suited for clinical application.

The present disclosure is not to be limited in scope by the specific options described herein. Indeed, various modifications of the present disclosure in addition to those described herein will become apparent to those skilled in the art from the foregoing description.

**LIST OF SEQUENCES:**

| **SEQ ID NO** | **SEQUENCE** | **DESCRIPTION** |
|---|---|---|
| 1 | | CD16V-BB-Z |
| | | |
| 2 | | V158 FCGR3A variant |
| 3 | | hinge and transmembrane domains of CD8alpha |
| 4 | KRGRKKLLYIFKQPFMRPVQTTQEEDGCSCRFPEEEEGGCEL | 4-1BB signaling domain |
| 5 | | CD3zeta signaling domain |
| 6 | MALPVTALLLPLALLLHAARP | signal peptide of CD8alpha |
| 7 | CTTCTGCAGGGGGCTTGTTGGGAGTAAAAATGTGTC | synthetic/ primer |
| 8 | GACACATTTTTACTCCCAACAAGCCCCCTGCAGAAG | synthetic/ primer |
| 9 | | CD16V-BB-Z |
| 10 | | V158 FCGR3A variant |
| | | |
| 11 | | hinge and transmembr ane domains of CD8alpha |
| 12 | | 4-1BB signaling domain |
| 13 | | CD3zeta signaling domain |
| 14 | | signal peptide of CD8alpha |
| 15 | | CD16F-BB-Z |
| 16 | | F158 FCGR3A variant |
| 17 | | CD16F-BB-Z |
| | | |
| 18 | | F158 FCGR3A variant |

## Claims

1. A chimeric receptor comprising: (a) an extracellular ligand-binding domain of F158 *FCGR3A* or the V158 *FCGR3A* variant; (b) a hinge and transmembrane domains of CD8α; and (c) a cytoplasmic domain comprising a 4-1BB signaling domain and a CD3ζ signaling domain.

2. The chimeric receptor of claim 1, wherein the chimeric receptor further comprises a signal peptide of CD8α; and/or,
optionally wherein the extracellular ligand-binding domain of F158 *FCGR3A* and the V158 *FCGR3A* variant consists of the amino acid sequence of SEQ ID NO:16 and SEQ ID NO: 2, respectively; and/or
optionally wherein the hinge and transmembrane domains of CD8α consist of the amino acid sequence of SEQ ID NO: 3; and/or
optionally wherein the 4-1 BB signaling domain consists of the amino acid sequence of SEQ ID NO: 4; and/or,
optionally wherein the CD3ζ signaling domain consists of the amino acid sequence of SEQ ID NO: 5; and/or
optionally wherein the signal peptide of CD8α consists of the amino acid sequence of SEQ ID NO: 6.

3. The chimeric receptor of claim 1, which comprises the amino acid sequence of residues 22-436 of SEQ ID NO: 1 or SEQ ID NO:15, or which comprises the amino acid sequence of SEQ ID NO: 1 or SEQ ID NO:15.

4. An isolated polynucleotide, comprising a nucleotide sequence encoding the chimeric receptor of any one of claims 1-3.

5. The isolated polynucleotide of claim 4, which comprises the nucleotide sequence of SEQ ID NO: 10 or SEO ID NO: 18; and/or
optionally wherein the isolated polynucleotide comprises the nucleotide sequence of SEQ ID NO: 11; and/or
optionally the isolated polynucleotide comprises the nucleotide sequence of SEQ ID NO: 12; and/or
optionally the isolated polynucleotide comprises the nucleotide sequence of SEQ ID NO: 13; and/or optionally the isolated polynucleotide comprises the nucleotide sequence of SEQ ID NO: 14.

6. The isolated polynucleotide of claim 4, which comprises the nucleotide sequence of SEQ ID NO: 9 or SEQ ID NO: 17.

7. A pharmaceutical composition comprising the polynucleotide of any one of claims 4-6 and a pharmaceutically acceptable carrier or excipient.

8. A vector comprising the polynucleotide of any one of claims 4-6;
optionally wherein the polynucleotide is operatively linked to at least one regulatory element for expression of the chimeric receptor encoded by the polynucleotide; and/or, optionally wherein the vector is a viral vector; and,
optionally wherein the viral vector is a retroviral vector or a lentiviral vector.

9. An isolated host cell, comprising the chimeric receptor of any one of claims 1-3; optionally wherein the host cell is a T lymphocyte or an NK cell; and,
optionally wherein the T lymphocyte or the NK cell is activated and/or expanded *ex vivo.*

10. The host cell of claim 9, wherein the T lymphocyte is activated in the presence of one or more agents selected from the group consisting of anti-CD3/CD28, IL-2, and phytohemoagglutinin, or wherein the NK cell is activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line; and/or,
optionally wherein the host cell is an autologous T lymphocyte or an autologous NK cell isolated from a patient having a cancer, or an allogeneic T lymphocyte or an allogeneic NK cell; and,
optionally wherein the endogenous T cell receptor in the allogeneic T lymphocyte has been inhibited or eliminated.

11. A pharmaceutical composition, comprising the vector of claim 8 or a host cell of any one of claims 9 or 10, and a pharmaceutically acceptable carrier or excipient;
optionally wherein the pharmaceutical composition further comprises an antibody which exerts cytotoxicity to cancer cells; and,
optionally wherein the antibody binds to cancer cells and has a human or humanized Fc portion that binds to human CD16, or wherein the antibody is selected from the group consisting of Rituximab, Trastuzumab, hu14.18K322A, Epratuzumab, Cetuximab, and Labetuzumab.

12. A T lymphocyte or NK cell expressing the chimeric receptor of any one of claims 1-3 for use in enhancing the efficacy of an antibody-based immunotherapy of a cancer in a subject in need thereof, wherein the subject is being treated with an antibody which binds to cancer cells, and wherein the subject is optionally administered with an effective amount of interleukin-2 (IL-2).

13. A T lymphocyte or NK cell expressing the chimeric receptor of any one of claims 1-3 for use in enhancing a T lymphocyte or a NK cell antibody-dependent cell cytotoxicity (ADCC) in a subject;
optionally wherein the subject is being treated with an antibody which binds to cancer cells; and/or,
optionally wherein the subject is a human patient having a cancer, and/or
optionally wherein the subject is administered an effective amount of interleukin-2 (IL-2).

14. The T lymphocyte or NK cell for use according to claim 12 or claim 13, wherein the antibody has a human or humanized Fc portion, which binds to human CD16, or wherein the antibody is selected from the group consisting of Rituximab, Trastuzumab, hu14, 18K322A, Epratuzumab, Cetuximab, and Labetuzumab; and/or
optionally wherein the cancer is selected from the group consisting of carcinoma, lymphoma, sarcoma, blastoma, and leukemia, or wherein the cancer is selected from the group consisting of a cancer of B-cell origin, breast cancer, gastric cancer, neuroblastoma, osteosarcoma, lung cancer, melanoma, prostate cancer, colon cancer, renal cell carcinoma, ovarian cancer, rhabdomyosarcoma, leukemia, and Hodgkin's lymphoma; and, optionally wherein the cancer of B-cell origin is selected from the group consisting of B-lineage acute lymphoblastic leukemia, B-cell chronic lymphocytic leukemia, and B-cell non-Hodgkin's lymphoma.

15. The T lymphocyte or NK cell for use according to any of claims 12 to 14, wherein the T lymphocytes or NK cells are autologous T lymphocytes or autologous NK cells isolated from the subject, the autologous T lymphocytes or NK cells being optionally activated and/or expanded *ex vivo* prior to administering to the subject; or wherein the T lynmphocytes or NK cells are allogeneic T lymphocytes, in which the expression of the endogenous T cell receptor has been optionally inhibited or eliminated, or allogenic NK cells; the allogenic T lymphocytes or NK cells being optionally activated and/or expanded *ex vivo* prior to administering to the subject.

16. The T lymphocyte or NK cell for use according to any of claims 12 to 15, wherein the chimeric receptor is introduced into the T lymphocytes or the NK cells by a method selected from the group consisting of retroviral transduction, lentiviral transduction, DNA electroporation, and RNA electroporation.

17. The T lymphocyte or NK cell for use according to claim 15 or claim 16, wherein the T lymphocyte is activated in the presence of one or more agents selected from the group consisting of anti-CD3/CD28, IL-2, and phytohaemoagglutinin, or wherein the NK cell is activated in the presence of one or more agents selected from the group consisting of CD137 ligand protein, CD137 antibody, IL-15 protein, IL-15 receptor antibody, IL-2 protein, IL-12 protein, IL-21 protein, and K562 cell line.

## Patentansprüche

1. Chimärer Rezeptor, Folgendes umfassend: (a) eine extrazelluläre Liganden bindende Domäne von F158 FCGR*3A* oder der Variante V158 FCGR*3A*; (b) eine Hinge- und mehrere Transmembrandomänen von CD8α; und (c) eine zytoplasmatische Domäne, umfassend eine 4-1BB-Signalisierungsdomäne und eine CD3ζ-Signalisierungsdomäne.

2. Chimärer Rezeptor nach Anspruch 1, wobei der chimäre Rezeptor ferner ein Signalpeptid von CD8α umfasst; und/oder
optional wobei die extrazelluläre Liganden bindende Domäne von F158 FCGR3A und der Variante V158 FCGR3A aus der Aminosäuresequenz von SEQ ID NO: 16 beziehungsweise SEQ ID NO: 2 besteht; und/oder
optional wobei die Hinge- und die Transmembrandomänen von CD8α aus der Aminosäuresequenz von SEQ ID NO: 3 bestehen;
optional wobei die 4-1BB-Signalisierungsdomäne aus der Aminosäuresequenz von SEQ ID NO: 4 besteht; und/oder
optional wobei die CD3-Signalisierungsdomäne aus der Aminosäuresequenz von SEQ ID NO: 5 besteht; und/oder
optional wobei das Signalpeptid von CD8α aus der Aminosäuresequenz von SEQ ID NO: 6 besteht.

3. Chimärer Rezeptor nach Anspruch 1, umfassend die Aminosäuresequenz der Reste 22-436 von SEQ ID NO: 1 oder SEQ ID NO: 15, oder die Aminosäuresequenz von SEQ ID NO: 1 oder SEQ ID NO: 15 umfassend.

4. Isoliertes Polynukleotid, umfassend die Nukleotidsequenz, die für den chimären Rezeptor nach einem der Ansprüche 1-3 codiert.

5. Isoliertes Polynukleotid nach Anspruch 4, umfassend die Nukleotidsequenz aus SEQ ID NO: 10 oder SEQ ID NO: 18; und/oder
optional wobei das isolierte Polynukleotid die Nukleotidsequenz von SEQ ID NO: 11 umfasst; und/oder
optional das isolierte Polynukleotid die Nukleotidsequenz von SEQ ID NO: 12 umfasst; und/oder
optional das isolierte Polynukleotid die Nukleotidsequenz von SEQ ID NO: 13 umfasst; und/oder
optional das isolierte Polynukleotid die Nukleotidsequenz von SEQ ID NO: 14 umfasst.

6. Isoliertes Polynukleotid nach Anspruch 4, umfassend die Nukleotidsequenz von SEQ ID NO: 9 oder SEQ ID NO: 17.

7. Pharmazeutische Zusammensetzung, umfassend das Polynukleotid nach einem der Ansprüche 4-6 und einen pharmazeutisch unbedenklichen Träger oder Hilfsstoff.

8. Vektor, umfassend das Polynukleotid nach einem der Ansprüche 4-6;
optional wobei das Polynukleotid wirkverbunden ist mit wenigstens einem regulatorischen Element zum Exprimieren des chimären Rezeptors, für den durch das Polynukleotid codiert wird; und/oder
optional wobei der Vektor ein viraler Vektor ist; und
optional wobei der virale Vektor ein retroviraler Vektor oder ein lentiviraler Vektor ist.

9. Isolierte Wirtszelle, umfassend den chimären Rezeptor nach einem der Ansprüche 1-3;
optional wobei die Wirtszelle ein T-Lymphozyt oder eine NK-Zelle ist; und
optional wobei der T-Lymphozyt *ex vivo* aktiviert oder expandiert ist.

10. Wirtszelle nach Anspruch 9, wobei der T-Lymphozyt in Gegenwart eines oder mehrerer Mittel aktiviert wird, ausgewählt aus der Gruppe bestehend aus Anti-CD3/CD28, IL-2 und Phytohämoagglutinin oder wobei die NK-Zelle in Gegenwart eines oder mehrerer Mittel aktiviert wird, ausgewählt aus der Gruppe bestehend aus CD137-Ligandenprotein, CD137-Antikörper, IL-15-Protein, IL-15-Rezeptorantikörper, IL-2-Pro, IL-12-Protein, IL-21-Protein und der Zelllinie K562; und/oder
optional wobei die Wirtszelle ein autologer T-Lymphozyt oder eine autologe NK-Zelle, isoliert aus einem Patienten mit einem Krebs, oder ein allogener T-Lymphozyt oder eine allogene NK-Zelle ist; und
optional wobei der endogene T-Zellen-Rezeptor in dem allogenen T-Lymphozyten gehemmt oder eliminiert worden ist.

11. Pharmazeutische Zusammensetzung, umfassend den Vektor nach Anspruch 8 oder eine Wirtszelle nach einem der Ansprüche 9 und 10 und einen pharmazeutisch unbedenklichen Träger oder Hilfsstoff;
optional wobei die pharmazeutische Zusammensetzung ferner einen Antikörper umfasst, der auf Krebszellen zytotoxisch wirkt; und
optional wobei der Antikörper an Krebszellen bindet und einen humanen oder humanisierten Fc-Abschnitt aufweist, der an humanes CD16 bindet, oder wobei der Antikörper ausgewählt ist aus der Gruppe bestehend aus: Rituximab, Trastuzumab, hu14.18K322A, Epratuzumab, Cetuximab und Labetuzumab.

12. T-Lymphozyt oder NK-Zelle, die den chimären Rezeptor nach einem der Ansprüche 1-3 exprimiert, für den Gebrauch beim Verbessern der Effizienz einer antikörperbasierten Immuntherapie eines Krebses in einem Subjekt, das dies benötigt, wobei das Subjekt mit einem Antikörper behandelt wird, der an Krebszellen bindet, und wobei dem Subjekt optional eine wirksame Menge Interleukin-2 (IL-2) verabreicht wird.

13. T-Lymphozyt oder NK-Zelle, die den chimären Rezeptor nach einem der Ansprüche 1-3 exprimiert, für den Gebrauch beim Verstärken der antikörperabhängigen zellvermittelten Zytotoxizität (antibody-dependent cell cytotoxicity - ADCC) für T-Lymphozyten oder NK-Zellen in einem Subjekt;
optional wobei das Subjekt mit einem Antikörper behandelt wird, das an Krebszellen bindet; und/oder
optional wobei das Subjekt ein menschlicher Patient ist, der einen Krebs hat, und/oder optional wobei dem Subjekt eine wirksame Menge Interleukin-2 (IL-2) verabreicht wird.

14. T-Lymphozyt oder NK-Zelle für den Gebrauch nach Anspruch 12 oder Anspruch 13, wobei der Antikörper einen humanen oder humanisierten Fc-Abschnitt aufweist, der an humanes CD16 bindet, oder wobei der Antikörper ausgewählt ist aus der Gruppe bestehend aus: Rituximab, Trastuzumab, hu14.18K322A, Epratuzumab, Cetuximab und Labetuzumab; und/oder
optional wobei der Krebs ausgewählt ist aus der Gruppe bestehend aus Karzinom, Lymphom, Sarkom, Blastom und Leukämie oder wobei der Krebs ausgewählt ist aus der Gruppe bestehend aus: einem Krebs mit B-Zellen-Ursprung, Brustkrebs, Magenkrebs, Neuroblastom, Osteosarkom, Lungenkrebs, Melanom, Prostatakrebs, Kolonkrebs, Nierenzellkarzinom, Eierstockkrebs, Rhabdomyosarkom, Leukämie und Hodgkin-Lymphom; und
optional wobei der Krebs mit B-Zellen-Ursprung ausgewählt ist aus der Gruppe bestehend aus akuter lymphatischer B-Zelllinien-Leukämie, chronischer lymphozytärer B-Zell-Leukämie und B-Zell-Non-Hodgin-Lymphom.

15. T-Lymphozyt oder NK-Zelle für den Gebrauch nach einem der Ansprüche 12 bis 14, wobei die T-Lymphozyten oder die NK-Zellen autologe T-Lymphozyten oder autologe NK-Zellen sind, die aus dem Subjekt isoliert worden sind, wobei die autologen T-Lymphozyten oder NK-Zellen vor dem Verabreichen an das Subjekt optional *ex vivo* aktiviert und/oder expandiert werden; oder wobei die T-Lymphozyten oder NK-Zellen allogene T-Lymphozyten, in denen die Exprimierung des endogenen T-Zell-Rezeptors optional gehemmt oder eliminiert worden ist, oder allogene NK-Zellen sind; wobei die allogenen T-Lymphozyten oder NK-Zellen vor dem Verabreichen an das Subjekt optional *ex vivo* aktiviert und/oder expandiert worden sind.

16. T-Lymphozyt oder NK-Zelle für den Gebrauch nach einem der Ansprüche 12 bis 15, wobei der chimäre Rezeptor über ein Verfahren, ausgewählt aus der Gruppe bestehend aus retroviraler Transduktion, lentiviraler Transduktion, DNA-Elektroporation und RNA-Elektroporation in die T-Lymphozyten oder die NK-Zellen eingeführt wird.

17. T-Lymphozyt oder NK-Zelle für den Gebrauch nach Anspruch 15 oder Anspruch 16, wobei der T-Lymphozyt in Gegenwart eines oder mehrerer Mittel aktiviert wird, ausgewählt aus der Gruppe bestehend aus Anti-CD3/CD28, IL-2 und Phytohämoagglutinin, oder wobei die NK-Zelle in Gegenwart eines oder mehrerer Mittel aktiviert wird, ausgewählt aus der Gruppe bestehend aus CD137-Ligandenprotein, CD137-Antikörper, IL-15-Protein, IL-15-Rezeptorantikörper, IL-2-Protein, IL-12-Protein, IL-21-Protein und der Zelllinie K562.

## Revendications

1. Récepteur chimérique comprenant : (a) un domaine extracellulaire de liaison au ligand du variant F158 *FCGR3A* ou du variant V158 *FCGR3A* ; (b) des domaines charnière et transmembranaire de CD8α ; et (c) un domaine cytoplasmique comprenant un domaine de signalisation 4-1 BB et un domaine de signalisation CD3ζ.

2. Récepteur chimérique selon la revendication 1, lequel récepteur chimérique comprend en outre un peptide signal de CD8α ; et/ou
facultativement où le domaine extracellulaire de liaison au ligand du variant F158 *FCGR3A* ou du variant V158 *FCGR3A* comprend la séquence d'acides aminés de SEQ ID n° : 16 et de SEQ ID n° : 2, respectivement ; et/ou
facultativement où les domaines charnière et transmembranaire de CD8α comprennent la séquence d'acides aminés de SEQ ID n° : 3 ; et/ou
facultativement où le domaine de signalisation 4-1 BB comprend la séquence d'acides aminés de SEQ ID n° : 4 ; et/ou
facultativement où le domaine de signalisation CD3ζ comprend la séquence d'acides aminés de SEQ ID n° : 5 ; et/ou
facultativement où le peptide signal de CD8α comprend la séquence d'acides aminés de SEQ ID n° : 6.

3. Récepteur chimérique selon la revendication 1, qui comprend la séquence d'acides aminés des résidus 22-436 de SEQ ID n° : 1 ou de SEQ ID n° : 15, ou qui comprend la séquence d'acides aminés de SEQ ID n° : 1 ou de SEQ ID n° : 15.

4. Polynucléotide isolé, comprenant une séquence nucléotidique codant pour le récepteur chimérique selon l'une quelconque des revendications 1 à 3.

5. Polynucléotide isolé selon la revendication 4, qui comprend la séquence nucléotidique de ID n° : 10 ou de ID n° : 18 ; et/ou
facultativement lequel polynucléotide isolé comprend la séquence nucléotidique de ID n° : 11 ; et/ou
facultativement lequel polynucléotide isolé comprend la séquence nucléotidique de ID n° : 12 ; et/ou
facultativement lequel polynucléotide isolé comprend la séquence nucléotidique de ID n° : 13 ; et/ou
facultativement lequel polynucléotide isolé comprend la séquence nucléotidique de ID n° : 14.

6. Polynucléotide isolé selon la revendication 4, qui comprend la séquence nucléotidique de ID n° : 9 ou de ID n° : 17.

7. Composition pharmaceutique comprenant le polynucléotide selon l'une quelconque des revendications 4 à 6 et un support ou excipient pharmaceutiquement acceptable.

8. Vecteur comprenant le polynucléotide selon l'une quelconque des revendications 4 à 6;
facultativement où le polynucléotide est opérationnellement lié à au moins un élément régulateur pour l'expression du récepteur chimérique codé par le polynucléotide ; et/ou
facultativement lequel vecteur est un vecteur viral ; et
facultativement lequel vecteur viral est un vecteur rétroviral ou un vecteur lentiviral.

9. Cellule hôte isolée, comprenant le récepteur chimérique selon l'une quelconque des revendications 1 à 3 ;
facultativement laquelle cellule hôte est un lymphocyte T ou une cellule NK ; et facultativement où le lymphocyte T ou la cellule NK est activé(e) et/ou expansé(e) *ex vivo.*

10. Cellule hôte selon la revendication 9, où le lymphocyte T est activé en présence d'un ou de plusieurs agents choisis dans le groupe constitué par anti-CD3/CD28, IL-2 et la phytohémagglutinine, ou la cellule NK est activée en présence d'un ou de plusieurs agents choisis dans le groupe constitué par la protéine du ligand CD137, l'anticorps CD137, la protéine IL-15, l'anticorps du récepteur de l'IL-15, la protéine IL-2, la protéine IL-12, la protéine IL-21 et la lignée cellulaire K562 ; et/ou
facultativement laquelle cellule hôte est un lymphocyte T autologue ou une cellule NK autologue isolé(e) d'un patient ayant un cancer, ou un lymphocyte T allogénique ou une cellule NK allogénique ; et
facultativement où le récepteur des lymphocytes T endogènes dans le lymphocyte T allogénique a été inhibé ou éliminé.

11. Composition pharmaceutique, comprenant le vecteur selon la revendication 8 ou une cellule hôte selon l'une quelconque des revendications 9 ou 10, et un support ou excipient pharmaceutiquement acceptable ;
facultativement laquelle composition pharmaceutique comprend en outre un anticorps qui exerce une cytotoxicité sur les cellules cancéreuses ; et
facultativement où l'anticorps se lie aux cellules cancéreuses et a une portion Fc humaine ou humanisée qui se lie au CD16 humain, ou l'anticorps est choisi dans le groupe constitué par le rituximab, le trastuzumab, hu14.18K322A, l'épratuzumab, le cétuximab et le labétuzumab.

12. Lymphocyte T ou cellule NK exprimant le récepteur chimérique selon l'une quelconque des revendications 1 à 3 pour une utilisation dans l'amélioration de l'efficacité d'une immunothérapie à base d'anticorps d'un cancer chez un patient en ayant besoin, lequel patient est traité avec un anticorps qui se lie aux cellules cancéreuses, et lequel patient reçoit facultativement une administration d'une quantité efficace d'interleukine-2 (IL-2).

13. Lymphocyte T ou cellule NK exprimant le récepteur chimérique selon l'une quelconque des revendications 1 à 3 pour une utilisation dans l'amélioration de la cytotoxicité cellulaire dépendante des anticorps (ADCC) d'un lymphocyte T ou d'une cellule NK chez un patient ;
facultativement où le patient est traité avec un anticorps qui se lie aux cellules cancéreuses ; et/ou
facultativement où le patient est un patient humain ayant un cancer ; et/ou
facultativement où le patient reçoit une administration d'une quantité efficace d'interleukine-2 (IL-2).

14. Lymphocyte T ou cellule NK pour une utilisation selon la revendication 12 ou 13, où l'anticorps a une portion Fc humaine ou humanisée qui se lie au CD16 humain, ou l'anticorps est choisi dans le groupe constitué par le rituximab, le trastuzumab, hu14.18K322A, l'épratuzumab, le cétuximab et le labétuzumab ; et/ou
facultativement où le cancer est choisi dans le groupe constitué par le carcinome, le lymphome, le sarcome, le blastome et la leucémie, ou le cancer est choisi dans le groupe constitué par un cancer d'immunotype B, le cancer du sein, le cancer de l'estomac, le neuroblastome, l'ostéosarcome, le cancer du poumon, le mélanome, le cancer de la prostate, le cancer du côlon, le carcinome à cellules rénales, le cancer de l'ovaire, le rhabdomyosarcome, la leucémie et le lymphome hodgkinien ; et
facultativement où le cancer d'immunotype B est choisi dans le groupe constitué par la leucémie aiguë lymphoblastique de la lignée B, la leucémie lymphoïde chronique des lymphocytes B et le lymphome non hodgkinien des lymphocytes B.

15. Lymphocyte T ou cellule NK pour une utilisation selon l'une quelconque des revendications 12 à 14, où les lymphocytes T ou les cellules NK sont des lymphocytes T autologues ou des cellules NK autologues isolé(e)s du patient, les lymphocytes T ou les cellules NK autologues étant facultativement activé(e)s et/ou expansé(e)s *ex vivo* avant l'administration au patient ; ou les lymphocytes T ou les cellules NK sont des lymphocytes T allogéniques dans lesquels l'expression du récepteur des lymphocytes T endogènes a été facultativement inhibée ou éliminée, ou des cellules NK allogéniques ; les lymphocytes T ou les cellules NK allogéniques étant facultativement activé(e)s et/ou expansé(e)s *ex vivo* avant l'administration au patient.

16. Lymphocyte T ou cellule NK pour une utilisation selon l'une quelconque des revendications 12 à 15, où le récepteur chimérique est introduit dans les lymphocytes T ou les cellules NK par un procédé choisi dans le groupe constitué par la transduction rétrovirale, la transduction lentivirale, l'électroporation d'ADN ou l'électroporation d'ARN.

17. Lymphocyte T ou cellule NK pour une utilisation selon la revendication 15 ou 16, où le lymphocyte T est activé en présence d'un ou de plusieurs agents choisis dans le groupe constitué par anti-CD3/CD28, IL-2 et la phytohémagglutinine, ou la cellule NK est activée en présence d'un ou de plusieurs agents choisis dans le groupe constitué par la protéine du ligand CD137, l'anticorps CD137, la protéine IL-15, l'anticorps du récepteur de l'IL-15, la protéine IL-2, la protéine IL-12, la protéine IL-21 et la lignée cellulaire K562.


### SEQUENCE LISTING

<110> National University of Singapore
<120> CHIMERIC RECEPTOR THAT TRIGGERS ANTIBODY-DEPENDENT CELL CYTOTOXICITY AGAINST MULTIPLE TUMORS
<130> N0565.70000WO00
<150> US 62/026,243
   <151> 2014-07-18
<150> US 61/892,218
   <151> 2013-10-17
<160> 18
<170> PatentIn version 3.5
<210> 1
   <211> 436
   <212> PRT
   <213> Artificial Sequence
<220>
   <223> Synthetic Polypeptide
<400> 1
   Met Ala Leu Pro Val Thr Ala Leu Leu Leu Pro Leu Ala Leu Leu Leu
   1               5                   10                  15
   His Ala Ala Arg Pro Gly Met Arg Thr Glu Asp Leu Pro Lys Ala Val
               20                  25                  30
   Val Phe Leu Glu Pro Gln Trp Tyr Arg Val Leu Glu Lys Asp Ser Val
           35                  40                  45
   Thr Leu Lys Cys Gln Gly Ala Tyr Ser Pro Glu Asp Asn Ser Thr Gln
       50                  55                  60
   Trp Phe His Asn Glu Ser Leu Ile Ser Ser Gln Ala Ser Ser Tyr Phe
   65                  70                  75                  80
   Ile Asp Ala Ala Thr Val Asp Asp Ser Gly Glu Tyr Arg Cys Gln Thr
                   85                  90                  95
   Asn Leu Ser Thr Leu Ser Asp Pro Val Gln Leu Glu Val His Ile Gly
               100                 105                 110
   Trp Leu Leu Leu Gln Ala Pro Arg Trp Val Phe Lys Glu Glu Asp Pro
           115                 120                 125
   Ile His Leu Arg Cys His Ser Trp Lys Asn Thr Ala Leu His Lys Val
       130                 135                 140
   Thr Tyr Leu Gln Asn Gly Lys Gly Arg Lys Tyr Phe His His Asn Ser
   145                 150                 155                 160
   Asp Phe Tyr Ile Pro Lys Ala Thr Leu Lys Asp Ser Gly Ser Tyr Phe
                   165                 170                 175
   Cys Arg Gly Leu Val Gly Ser Lys Asn Val Ser Ser Glu Thr Val Asn
               180                 185                 190
   Ile Thr Ile Thr Gln Gly Leu Ala Val Ser Thr Ile Ser Ser Phe Phe
           195                 200                 205
   Pro Pro Gly Tyr Gln Thr Thr Thr Pro Ala Pro Arg Pro Pro Thr Pro
       210                 215                 220
   Ala Pro Thr Ile Ala Ser Gln Pro Leu Ser Leu Arg Pro Glu Ala Cys
   225                 230                 235                 240
   Arg Pro Ala Ala Gly Gly Ala Val His Thr Arg Gly Leu Asp Phe Ala
                   245                 250                 255
   Cys Asp Ile Tyr Ile Trp Ala Pro Leu Ala Gly Thr Cys Gly Val Leu
               260                 265                 270
   Leu Leu Ser Leu Val Ile Thr Leu Tyr Cys Lys Arg Gly Arg Lys Lys
           275                 280                 285
   Leu Leu Tyr Ile Phe Lys Gln Pro Phe Met Arg Pro Val Gln Thr Thr
       290                 295                 300
   Gln Glu Glu Asp Gly Cys Ser Cys Arg Phe Pro Glu Glu Glu Glu Gly
   305                 310                 315                 320
   Gly Cys Glu Leu Arg Val Lys Phe Ser Arg Ser Ala Asp Ala Pro Ala
                   325                 330                 335
   Tyr Gln Gln Gly Gln Asn Gln Leu Tyr Asn Glu Leu Asn Leu Gly Arg
               340                 345                 350
   Arg Glu Glu Tyr Asp Val Leu Asp Lys Arg Arg Gly Arg Asp Pro Glu
           355                 360                 365
   Met Gly Gly Lys Pro Arg Arg Lys Asn Pro Gln Glu Gly Leu Tyr Asn
       370                 375                 380
   Glu Leu Gln Lys Asp Lys Met Ala Glu Ala Tyr Ser Glu Ile Gly Met
   385                 390                 395                 400
   Lys Gly Glu Arg Arg Arg Gly Lys Gly His Asp Gly Leu Tyr Gln Gly
                   405                 410                 415
   Leu Ser Thr Ala Thr Lys Asp Thr Tyr Asp Ala Leu His Met Gln Ala
               420                 425                 430
   Leu Pro Pro Arg
           435
<210> 2
   <211> 192
   <212> PRT
   <213> Artificial Sequence
<220>
   <223> Synthetic Polypeptide
<400> 2
   Gly Met Arg Thr Glu Asp Leu Pro Lys Ala Val Val Phe Leu Glu Pro
   1               5                   10                  15
   Gln Trp Tyr Arg Val Leu Glu Lys Asp Ser Val Thr Leu Lys Cys Gln
               20                  25                  30
   Gly Ala Tyr Ser Pro Glu Asp Asn Ser Thr Gln Trp Phe His Asn Glu
           35                  40                  45
   Ser Leu Ile Ser Ser Gln Ala Ser Ser Tyr Phe Ile Asp Ala Ala Thr
       50                  55                  60
   Val Asp Asp Ser Gly Glu Tyr Arg Cys Gln Thr Asn Leu Ser Thr Leu
   65                  70                  75                  80
   Ser Asp Pro Val Gln Leu Glu Val His Ile Gly Trp Leu Leu Leu Gln
                   85                  90                  95
   Ala Pro Arg Trp Val Phe Lys Glu Glu Asp Pro Ile His Leu Arg Cys
               100                 105                 110
   His Ser Trp Lys Asn Thr Ala Leu His Lys Val Thr Tyr Leu Gln Asn
           115                 120                 125
   Gly Lys Gly Arg Lys Tyr Phe His His Asn Ser Asp Phe Tyr Ile Pro
       130                 135                 140
   Lys Ala Thr Leu Lys Asp Ser Gly Ser Tyr Phe Cys Arg Gly Leu Val
   145                 150                 155                 160
   Gly Ser Lys Asn Val Ser Ser Glu Thr Val Asn Ile Thr Ile Thr Gln
                   165                 170                 175
   Gly Leu Ala Val Ser Thr Ile Ser Ser Phe Phe Pro Pro Gly Tyr Gln
               180                 185                 190
<210> 3
   <211> 69
   <212> PRT
   <213> Artificial Sequence
<220>
   <223> Synthetic Polypeptide
<400> 3
   Thr Thr Thr Pro Ala Pro Arg Pro Pro Thr Pro Ala Pro Thr Ile Ala
   1               5                   10                  15
   Ser Gln Pro Leu Ser Leu Arg Pro Glu Ala Cys Arg Pro Ala Ala Gly
               20                  25                  30
   Gly Ala Val His Thr Arg Gly Leu Asp Phe Ala Cys Asp Ile Tyr Ile
           35                  40                  45
   Trp Ala Pro Leu Ala Gly Thr Cys Gly Val Leu Leu Leu Ser Leu Val
       50                  55                  60
   Ile Thr Leu Tyr Cys
   65
<210> 4
   <211> 42
   <212> PRT
   <213> Artificial Sequence
<220>
   <223> Synthetic Polypeptide
<400> 4
   Lys Arg Gly Arg Lys Lys Leu Leu Tyr Ile Phe Lys Gln Pro Phe Met
   1               5                   10                  15
   Arg Pro Val Gln Thr Thr Gln Glu Glu Asp Gly Cys Ser Cys Arg Phe
               20                  25                  30
   Pro Glu Glu Glu Glu Gly Gly Cys Glu Leu
           35                  40
<210> 5
   <211> 112
   <212> PRT
   <213> Artificial Sequence
<220>
   <223> Synthetic Polypeptide
<400> 5
   Arg Val Lys Phe Ser Arg Ser Ala Asp Ala Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 6
   <211> 21
   <212> PRT
   <213> Artificial Sequence
<220>
   <223> Synthetic Polypeptide
<400> 6
   Met Ala Leu Pro Val Thr Ala Leu Leu Leu Pro Leu Ala Leu Leu Leu
   1               5                   10                  15
   His Ala Ala Arg Pro
               20
<210> 7
   <211> 36
   <212> DNA
   <213> Artificial Sequence
<220>
   <223> Synthetic Polynucleotide
<400> 7
   cttctgcagg gggcttgttg ggagtaaaaa tgtgtc 36
<210> 8
   <211> 36
   <212> DNA
   <213> Artificial Sequence
<220>
   <223> Synthetic Polynucleotide
<400> 8
   gacacatttt tactcccaac aagccccctg cagaag 36
<210> 9
   <211> 1311
   <212> DNA
   <213> Artificial Sequence
<220>
   <223> Synthetic Polynucleotide
<400> 9
   atggccttac cagtgaccgc cttgctcctg ccgctggcct tgctgctcca cgccgccagg      60
   ccgggcatgc ggactgaaga tctcccaaag gctgtggtgt tcctggagcc tcaatggtac     120
   agggtgctcg agaaggacag tgtgactctg aagtgccagg gagcctactc ccctgaggac     180
   aattccacac agtggtttca caatgagagc ctcatctcaa gccaggcctc gagctacttc     240
   attgacgctg ccacagtcga cgacagtgga gagtacaggt gccagacaaa cctctccacc     300
   ctcagtgacc cggtgcagct agaagtccat atcggctggc tgttgctcca ggcccctcgg     360
   tgggtgttca aggaggaaga ccctattcac ctgaggtgtc acagctggaa gaacactgct     420
   ctgcataagg tcacatattt acagaatggc aaaggcagga agtattttca tcataattct     480
   gacttctaca ttccaaaagc cacactcaaa gacagcggct cctacttctg cagggggctt     540
   gttgggagta aaaatgtgtc ttcagagact gtgaacatca ccatcactca aggtttggca     600
   gtgtcaacca tctcatcatt ctttccacct gggtaccaaa ccacgacgcc agcgccgcga     660
   ccaccaacac cggcgcccac catcgcgtcg cagcccctgt ccctgcgccc agaggcgtgc     720
   cggccagcgg cggggggcgc agtgcacacg agggggctgg acttcgcctg tgatatctac     780
   atctgggcgc ccttggccgg gacttgtggg gtccttctcc tgtcactggt tatcaccctt     840
   tactgcaaac ggggcagaaa gaaactcctg tatatattca aacaaccatt tatgagacca     900
   gtacaaacta ctcaagagga agatggctgt agctgccgat ttccagaaga agaagaagga     960
   ggatgtgaac tgagagtgaa gttcagcagg agcgcagacg cccccgcgta ccagcagggc    1020
   cagaaccagc tctataacga gctcaatcta ggacgaagag aggagtacga tgttttggac    1080
   aagagacgtg gccgggaccc tgagatgggg ggaaagccga gaaggaagaa ccctcaggaa    1140
   ggcctgtaca atgaactgca gaaagataag atggcggagg cctacagtga gattgggatg    1200
   aaaggcgagc gccggagggg caaggggcac gatggccttt accagggtct cagtacagcc    1260
   accaaggaca cctacgacgc ccttcacatg caggccctgc cccctcgcta a             1311
<210> 10
   <211> 576
   <212> DNA
   <213> Artificial Sequence
<220>
   <223> Synthetic Polynucleotide
<400> 10
   ggcatgcgga ctgaagatct cccaaaggct gtggtgttcc tggagcctca atggtacagg        60
   gtgctcgaga aggacagtgt gactctgaag tgccagggag cctactcccc tgaggacaat       120
   tccacacagt ggtttcacaa tgagagcctc atctcaagcc aggcctcgag ctacttcatt       180
   gacgctgcca cagtcgacga cagtggagag tacaggtgcc agacaaacct ctccaccctc       240
   agtgacccgg tgcagctaga agtccatatc ggctggctgt tgctccaggc ccctcggtgg       300
   gtgttcaagg aggaagaccc tattcacctg aggtgtcaca gctggaagaa cactgctctg       360
   cataaggtca catatttaca gaatggcaaa ggcaggaagt attttcatca taattctgac       420
   ttctacattc caaaagccac actcaaagac agcggctcct acttctgcag ggggcttgtt       480
   gggagtaaaa atgtgtcttc agagactgtg aacatcacca tcactcaagg tttggcagtg       540
   tcaaccatct catcattctt tccacctggg taccaa                                 576
<210> 11
   <211> 207
   <212> DNA
   <213> Artificial Sequence
<220>
   <223> Synthetic Polynucleotide
<400> 11
   accacgacgc cagcgccgcg accaccaaca ccggcgccca ccatcgcgtc gcagcccctg        60
   tccctgcgcc cagaggcgtg ccggccagcg gcggggggcg cagtgcacac gagggggctg       120
   gacttcgcct gtgatatcta catctgggcg cccttggccg ggacttgtgg ggtccttctc       180
   ctgtcactgg ttatcaccct ttactgc                                           207
<210> 12
   <211> 126
   <212> DNA
   <213> Artificial Sequence
<220>
   <223> Synthetic Polynucleotide
<400> 12
   aaacggggca gaaagaaact cctgtatata ttcaaacaac catttatgag accagtacaa 60
   actactcaag aggaagatgg ctgtagctgc cgatttccag aagaagaaga aggaggatgt 120
   gaactg 126
<210> 13
   <211> 339
   <212> DNA
   <213> Artificial Sequence
<220>
   <223> Synthetic Polynucleotide
<400> 13
   agagtgaagt tcagcaggag cgcagacgcc cccgcgtacc agcagggcca gaaccagctc 60
   tataacgagc tcaatctagg acgaagagag gagtacgatg ttttggacaa gagacgtggc 120
   cgggaccctg agatgggggg aaagccgaga aggaagaacc ctcaggaagg cctgtacaat 180
   gaactgcaga aagataagat ggcggaggcc tacagtgaga ttgggatgaa aggcgagcgc 240
   cggaggggca aggggcacga tggcctttac cagggtctca gtacagccac caaggacacc 300
   tacgacgccc ttcacatgca ggccctgccc cctcgctaa 339
<210> 14
   <211> 63
   <212> DNA
   <213> Artificial Sequence
<220>
   <223> Synthetic Polynucleotide
<400> 14
   atggccttac cagtgaccgc cttgctcctg ccgctggcct tgctgctcca cgccgccagg 60
   ccg 63
<210> 15
   <211> 436
   <212> PRT
   <213> Artificial Sequence
<220>
   <223> Synthetic Polypeptide
<400> 15
   Met Ala Leu Pro Val Thr Ala Leu Leu Leu Pro Leu Ala Leu Leu Leu
   1               5                   10                  15
   His Ala Ala Arg Pro Gly Met Arg Thr Glu Asp Leu Pro Lys Ala Val
               20                  25                  30
   Val Phe Leu Glu Pro Gln Trp Tyr Arg Val Leu Glu Lys Asp Ser Val
           35                  40                  45
   Thr Leu Lys Cys Gln Gly Ala Tyr Ser Pro Glu Asp Asn Ser Thr Gln
       50                  55                  60
   Trp Phe His Asn Glu Ser Leu Ile Ser Ser Gln Ala Ser Ser Tyr Phe
   65                  70                  75                  80
   Ile Asp Ala Ala Thr Val Asp Asp Ser Gly Glu Tyr Arg Cys Gln Thr
                   85                  90                  95
   Asn Leu Ser Thr Leu Ser Asp Pro Val Gln Leu Glu Val His Ile Gly
               100                 105                 110
   Trp Leu Leu Leu Gln Ala Pro Arg Trp Val Phe Lys Glu Glu Asp Pro
           115                 120                 125
   Ile His Leu Arg Cys His Ser Trp Lys Asn Thr Ala Leu His Lys Val
       130                 135                 140
   Thr Tyr Leu Gln Asn Gly Lys Gly Arg Lys Tyr Phe His His Asn Ser
   145                 150                 155                 160
   Asp Phe Tyr Ile Pro Lys Ala Thr Leu Lys Asp Ser Gly Ser Tyr Phe
                   165                 170                 175
   Cys Arg Gly Leu Phe Gly Ser Lys Asn Val Ser Ser Glu Thr Val Asn
               180                 185                 190
   Ile Thr Ile Thr Gln Gly Leu Ala Val Ser Thr Ile Ser Ser Phe Phe
           195                 200                 205
   Pro Pro Gly Tyr Gln Thr Thr Thr Pro Ala Pro Arg Pro Pro Thr Pro
       210                 215                 220
   Ala Pro Thr Ile Ala Ser Gln Pro Leu Ser Leu Arg Pro Glu Ala Cys
   225                 230                 235                 240
   Arg Pro Ala Ala Gly Gly Ala Val His Thr Arg Gly Leu Asp Phe Ala
                   245                 250                 255
   Cys Asp Ile Tyr Ile Trp Ala Pro Leu Ala Gly Thr Cys Gly Val Leu
               260                 265                 270
   Leu Leu Ser Leu Val Ile Thr Leu Tyr Cys Lys Arg Gly Arg Lys Lys
           275                 280                 285
   Leu Leu Tyr Ile Phe Lys Gln Pro Phe Met Arg Pro Val Gln Thr Thr
       290                 295                 300
   Gln Glu Glu Asp Gly Cys Ser Cys Arg Phe Pro Glu Glu Glu Glu Gly
   305                 310                 315                 320
   Gly Cys Glu Leu Arg Val Lys Phe Ser Arg Ser Ala Asp Ala Pro Ala
                   325                 330                 335
   Tyr Gln Gln Gly Gln Asn Gln Leu Tyr Asn Glu Leu Asn Leu Gly Arg
               340                 345                 350
   Arg Glu Glu Tyr Asp Val Leu Asp Lys Arg Arg Gly Arg Asp Pro Glu
           355                 360                 365
   Met Gly Gly Lys Pro Arg Arg Lys Asn Pro Gln Glu Gly Leu Tyr Asn
       370                 375                 380
   Glu Leu Gln Lys Asp Lys Met Ala Glu Ala Tyr Ser Glu Ile Gly Met
   385                 390                 395                 400
   Lys Gly Glu Arg Arg Arg Gly Lys Gly His Asp Gly Leu Tyr Gln Gly
                   405                 410                 415
   Leu Ser Thr Ala Thr Lys Asp Thr Tyr Asp Ala Leu His Met Gln Ala
               420                 425                 430
   Leu Pro Pro Arg
           435
<210> 16
   <211> 192
   <212> PRT
   <213> Artificial Sequence
<220>
   <223> Synthetic Polypeptide
<400> 16
   Gly Met Arg Thr Glu Asp Leu Pro Lys Ala Val Val Phe Leu Glu Pro
   1 5 10 15
   Gln Trp Tyr Arg Val Leu Glu Lys Asp Ser Val Thr Leu Lys Cys Gln
   20 25 30
   Gly Ala Tyr Ser Pro Glu Asp Asn Ser Thr Gln Trp Phe His Asn Glu
   35 40 45
   Ser Leu Ile Ser Ser Gln Ala Ser Ser Tyr Phe Ile Asp Ala Ala Thr
   50 55 60
   Val Asp Asp Ser Gly Glu Tyr Arg Cys Gln Thr Asn Leu Ser Thr Leu
   65 70 75 80
   Ser Asp Pro Val Gln Leu Glu Val His Ile Gly Trp Leu Leu Leu Gln
   85 90 95
   Ala Pro Arg Trp Val Phe Lys Glu Glu Asp Pro Ile His Leu Arg Cys
   100 105 110
   His Ser Trp Lys Asn Thr Ala Leu His Lys Val Thr Tyr Leu Gln Asn
   115 120 125
   Gly Lys Gly Arg Lys Tyr Phe His His Asn Ser Asp Phe Tyr Ile Pro
   130 135 140
   Lys Ala Thr Leu Lys Asp Ser Gly Ser Tyr Phe Cys Arg Gly Leu Phe
   145 150 155 160
   Gly Ser Lys Asn Val Ser Ser Glu Thr Val Asn Ile Thr Ile Thr Gln
   165 170 175
   Gly Leu Ala Val Ser Thr Ile Ser Ser Phe Phe Pro Pro Gly Tyr Gln
   180 185 190
<210> 17
   <211> 1311
   <212> DNA
   <213> Artificial Sequence
<220>
   <223> Synthetic Polynucleotide
<400> 17
   atggccttac cagtgaccgc cttgctcctg ccgctggcct tgctgctcca cgccgccagg 60
   ccgggcatgc ggactgaaga tctcccaaag gctgtggtgt tcctggagcc tcaatggtac 120
   agggtgctcg agaaggacag tgtgactctg aagtgccagg gagcctactc ccctgaggac 180
   aattccacac agtggtttca caatgagagc ctcatctcaa gccaggcctc gagctacttc 240
   attgacgctg ccacagtcga cgacagtgga gagtacaggt gccagacaaa cctctccacc 300
   ctcagtgacc cggtgcagct agaagtccat atcggctggc tgttgctcca ggcccctcgg 360
   tgggtgttca aggaggaaga ccctattcac ctgaggtgtc acagctggaa gaacactgct 420
   ctgcataagg tcacatattt acagaatggc aaaggcagga agtattttca tcataattct 480
   gacttctaca ttccaaaagc cacactcaaa gacagcggct cctacttctg cagggggctt 540
   tttgggagta aaaatgtgtc ttcagagact gtgaacatca ccatcactca aggtttggca 600
   gtgtcaacca tctcatcatt ctttccacct gggtaccaaa ccacgacgcc agcgccgcga 660
   ccaccaacac cggcgcccac catcgcgtcg cagcccctgt ccctgcgccc agaggcgtgc 720
   cggccagcgg cggggggcgc agtgcacacg agggggctgg acttcgcctg tgatatctac 780
   atctgggcgc ccttggccgg gacttgtggg gtccttctcc tgtcactggt tatcaccctt 840
   tactgcaaac ggggcagaaa gaaactcctg tatatattca aacaaccatt tatgagacca 900
   gtacaaacta ctcaagagga agatggctgt agctgccgat ttccagaaga agaagaagga 960
   ggatgtgaac tgagagtgaa gttcagcagg agcgcagacg cccccgcgta ccagcagggc 1020
   cagaaccagc tctataacga gctcaatcta ggacgaagag aggagtacga tgttttggac 1080
   aagagacgtg gccgggaccc tgagatgggg ggaaagccga gaaggaagaa ccctcaggaa 1140
   ggcctgtaca atgaactgca gaaagataag atggcggagg cctacagtga gattgggatg 1200
   aaaggcgagc gccggagggg caaggggcac gatggccttt accagggtct cagtacagcc 1260
   accaaggaca cctacgacgc ccttcacatg caggccctgc cccctcgcta a 1311
<210> 18
   <211> 576
   <212> DNA
   <213> Artificial Sequence
<220>
   <223> Synthetic Polynucleotide
<400> 18
   ggcatgcgga ctgaagatct cccaaaggct gtggtgttcc tggagcctca atggtacagg    60
   gtgctcgaga aggacagtgt gactctgaag tgccagggag cctactcccc tgaggacaat   120
   tccacacagt ggtttcacaa tgagagcctc atctcaagcc aggcctcgag ctacttcatt   180
   gacgctgcca cagtcgacga cagtggagag tacaggtgcc agacaaacct ctccaccctc   240
   agtgacccgg tgcagctaga agtccatatc ggctggctgt tgctccaggc ccctcggtgg   300
   gtgttcaagg aggaagaccc tattcacctg aggtgtcaca gctggaagaa cactgctctg   360
   cataaggtca catatttaca gaatggcaaa ggcaggaagt attttcatca taattctgac   420
   ttctacattc caaaagccac actcaaagac agcggctcct acttctgcag ggggcttttt   480
   gggagtaaaa atgtgtcttc agagactgtg aacatcacca tcactcaagg tttggcagtg   540
   tcaaccatct catcattctt tccacctggg taccaa                             576